# EUROPEAN PATENT APPLICATION

(11) **EP 3 960 848 A1**
(43) Date of publication of application: **02.03.2022**
(21) Application number: 20796218.4
(22) Date of filing: 23.04.2020
(51) Int. Cl.: C12N 5/077, A61K 35/34

(54) **METHOD FOR REGULATION OF SELECTIVE DIFFERENTIATION OF MUSCULOSKELETAL STEM CELLS**

(30) Priority: 23.04.2019 KR 20190047064
(71) Applicant: Cellatoz Therapeutics, Inc., Seongnam-si, Gyeonggi-do 13488 (KR)
(72) Inventor: HAN, Myung-Kwan, Jeonju-si Jeollabuk-do 54955 (KR)
(74) Representative: V.O.
(86) International application number: PCT/KR2020/005366
(87) International publication number: WO 2020/218845

(57) **Abstract**

The present disclosure is novel musculoskeletal stem cells (MSSCs) derived from embryonic stem cells (ESCs) or induced pluripotent stem cells (iPSCs), and a medium composition for selectively differentiation into bone, tooth, cartilage, ligament and muscle, and a method for inducing differentiation thereof.

## Description

### [Technical Field]

The present disclosure relates to a musculoskeletal stem cell capable of differentiating into various musculoskeletal tissues and a method for regulating the same to differentiate selective into muscle, ligament, cartilage, bone or tooth tissue.

### [Background Art]

Musculoskeletal diseases are diseases occurring in the musculoskeletal system. They may be classified depending on the parts where they occur, such as bone, teeth, muscles, ligaments, nerves, joints or other connective tissues. The disease collectively refers to health problems caused by overuse of force, repetitive movement, inappropriate working posture, physical contact with sharp edge, vibration, temperature, etc. (Buckwalter et al., 1993). Senescence leads to changes in the musculoskeletal system. Muscles decline in mass and function, and the cartilage of the joint also undergoes degenerative changes, resulting in arthritic pain and limited motion. In addition, the decrease in bone formation as compared to bone resorption in bone tissue may cause osteoporosis and fracture (Jae Suk Chang, 2009).

In 1988, Owen et al. first found out that bone marrow-derived mesenchymal stem cells can differentiate into bone cells, chondrocyte, muscle cells, adipocytes, fibrous cells, ligament and tendon through in vitro culture under specific conditions and, thereby, presented the possibility of use in regenerative medicine (Owen M. J. Cell Sci. Suppl. 1988; 10: 63-76). However, despite many clinical attempts, the treatment of regenerating mesenchymal stem cells directly to musculoskeletal tissue has not been successful (ClinicalTrials.gov).

For stem cell therapy for skeletal muscle damage, transplantation of skeletal muscle-derived stem cells such as satellite cells that different directly into muscle fiber or tissue engineering techniques using scaffolds have been attempted. However, although the satellite cells can proliferate in vitro, it is reported that their ability to proliferate, differentiate and regenerate is low when injected into muscle due to short culture period (NA Dumont et al. Compr. Physiol. 5(30); 1027-1059; 2015).

Recently, it has been verified that skeletal stem cells having totally different characteristics from mesenchymal stem cells found in mouse and human are involved in the regeneration of bone and cartilage and that they can be used for bone regeneration (Chan CKF et al., Cell 175(1); 43-56; 2018).

In a previous patent application (Korean Patent Application No. 10-2018-0127501), the inventors of the present disclosure have developed a method for inducing pluripotent stem cells such as embryonic stem cells and induced pluripotent stem cells to musculoskeletal stem cells that can differentiate into bone, muscle, cartilage, adipose, tendon, ligament, etc. Since the musculoskeletal stem cells can be induced from embryonic stem cells or induced pluripotent stem cells that can proliferate without limitation and the cells can be cultured for 10 passages or longer, they are suitable for regeneration of musculoskeletal tissue in patients with musculoskeletal diseases. For effective use of the musculoskeletal stem cells in treatment of musculoskeletal diseases, development of a method for inducing selective differentiation into bone, tooth, muscle, cartilage, tendon, ligament, etc. is necessary.

The above information described in the background section is only for enhancing the understanding of the background of the present disclosure and, therefore, it may contain information that does not form the prior art that is already known to those having ordinary knowledge in the art.

### [Disclosure]

### [Technical Problem]

The inventors of the present disclosure have identified that human musculoskeletal stem cells (hMSSCs) can be induced from human embryonic stem cells (hESCs) or human induced pluripotent stem cells (hiPSCs) and that the musculoskeletal stem cells can differentiate into bone through endochondral ossification. Furthermore, they have found out that the musculoskeletal stem cells can be differentiated selectively into musculoskeletal tissues such as tooth, cartilage, tendon, ligament, muscle, etc. in addition to bone, and have completed the present disclosure.

The present disclosure is directed to providing a medium composition for inducing differentiation into musculoskeletal stem cells.

The present disclosure is also directed to providing a method for preparing musculoskeletal stem cells, which includes a step of culturing ESCs or iPSCs in the medium.

The present disclosure is also directed to providing musculoskeletal stem cells differentiated from ESCs or iPSCs.

The present disclosure is also directed to providing a pharmaceutical composition for preventing or treating a musculoskeletal disease, which contains the musculoskeletal stem cells.

The present disclosure is also directed to providing a method for screening the musculoskeletal stem cells.

The present disclosure is also directed to providing a medium composition for inducing differentiation, which induces selective differentiation from the musculoskeletal stem cell into muscle, cartilage, tendon, ligament, bone or tooth.

The present disclosure is also directed to providing a method for inducing selective differentiation from the musculoskeletal stem cell into muscle, cartilage, tendon, ligament, bone or tooth.

The present disclosure is also directed to providing a method for treating damaged tendon or ligament, which includes a step of administering the musculoskeletal stem cells to a damaged tendon or ligament area of a subject.

The present disclosure is also directed to providing a method for treating arthritis, which includes a step of administering the musculoskeletal stem cells into the articular cavity of a subject.

Other objects and advantages of the present disclosure will become more apparent by the following detailed description, claims and drawings.

### [Technical Solution]

In an aspect of the present disclosure, the present disclosure provides a medium composition for inducing differentiation into musculoskeletal stem cells (MSSCs), which contains noggin, leukemia inhibitory factor (LIF), basic fibroblast growth factor (bFGF), Wnt signaling activator, extracellular signal-regulated kinase (ERK) signaling inhibitor and TGF-β/activin/nodal signaling inhibitor.

The inventors of the present disclosure have found an optimum medium composition that can induce musculoskeletal stem cells from human embryonic stem cells or human induced pluripotent stem cells and completed the present disclosure by identifying that the musculoskeletal stem cells can differentiate into bone through endochondral ossification and also into tooth, cartilage, tendon, ligament, muscle, etc.

In the present specification, the term "stem cell" refers to an undifferentiated cell that can differentiate into various body tissues. The stem cells can be classified into totipotent stem cells, pluripotent stem cells, multipotent stem cells, etc. The term stem cell may be used interchangeably with the terms such as precursor cell, progenitor cell, etc. In the present disclosure, the stem cells may be embryonic stem cells (ESCs), induced pluripotent stem cell (iPSCs) or mesenchymal stem cells (MSCs). Accordingly, the medium composition of the present disclosure may be used to induce differentiation of musculoskeletal stem cells using embryonic stem cells, induced pluripotent stem cells, etc.

The embryonic stem cell refers to a cell having pluripotency, which means the ability of the embryonic stem cell including proliferation without transformation, limitless proliferation, self-renewal and potential to differentiate into any of three germ layers, although not being limited thereto.

In the present specification, the term "musculoskeletal stem cell" refers to a cell that can differentiate into bone, cartilage, tendon, ligament, tooth, adipose and/or muscle without limitation.

The term "differentiation" refers to the specialization of the structure or function of a cell during cell division and proliferation, i.e., the process in which the shape or function of the cell, tissue, etc. of an organism is changed to perform given tasks. In general, it refers to a phenomenon in which a relatively simple system is divided into two or more qualitatively different subsystems. For example, the occurrence of qualitative difference in parts of an organism which has been almost homogeneous, such as the distinction between head and body from the initially homogeneous egg during ontogenesis or distinction between muscle cells and nerve cells, etc., which leads to qualitatively distinguishable parts or subsystems, is called differentiation.

The embryonic stem cells or induced pluripotent stem cells used in the present specification are derived from human, cow, horse, goat, sheep, dog, cat, mouse, rat, birds, etc., specifically from human.

In the present specification, the term "subject" refers to a subject to which the musculoskeletal stem cells of the present disclosure or cells differentiated therefrom are to be administered, and includes human, mammals such as cow, horse, goat, sheep, dog, cat, mouse, rat, etc., birds, reptiles, amphibians and fish.

The Wnt signaling activator of the present disclosure is specifically SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), kenpaullone (9-bromo-7,12-dihydro-indolo[3,2-d]-[1]benzazepin-6(5H)-one), CHIR99021 (9-bromo-7,12-dihydro-pyrido[3',2':2,3]azepino[4,5-b]indol-6(5H)-one), CP21R7 (3-(3-amino-phenyl)-4-(1-methyl-1H-indol-3-yl)-pyrrole-2,5-dione), SB203580 (4-(4-fluorophenyl)-2-(4-methylsulfinylphenyl)-5-(4-pyridyl)-1H-imidazole), H-89 (5-isoquinolinesulfonamide), purmorphamine (2-(1-naphthoxy)-6-(4-morpholinoanilino)-9-cyclohexylpurine) or IQ-1 (2-(4-acetyl-phenylazo)-2-[3,3-dimethyl-3,4-dihydro-2H-isoquinolin-(1E)-ylidene]-acet amide), although not being limited thereto.

The ERK signaling inhibitor of the present disclosure is specifically AS703026 (N-[(2S)-2,3-dihydroxypropyl]-3-[(2-fluoro-4-iodophenyl)amino]-isonicotinamide), AZD6244 (6-(4-bromo-2-chloroanilino)-7-fluoro-N-(2-hydroxyethoxy)-3-methylbenzimidazole-5-carboxamide), PD0325901 (N-[(2R)-2,3-dihydroxypropoxy]-3,4-difluoro-2-[(2-fluoro-4-iodophenyl)amino]-benza mide), ARRY-438162 (5-[(4-bromo-2-fluorophenyl)amino]-4-fluoro-N-(2-hydroxyethoxy)-1-methyl-1H-benzi midazole-6-carboxamide), RDEA119 ((S)-N-(3,4-difluoro-2-((2-fluoro-4-iodophenyl)amino)-6-methoxyphenyl)-1-(2,3-dihydr oxypropyl)cyclopropane-1-sulfonamide), GDC0973 ([3,4-difluoro-2-(2-fluoro-4-iodoanilino)phenyl]-3-hydroxy-3-[(2S)-piperidin-2-yl]-azetid in-1-yl-methanone), TAK-733 ((R)-3-(2,3-dihydroxypropyl)-6-fluoro-5-(2-fluoro-4-iodophenylamino)-8-methylpyrido[2,3-d]pyrimidine-4,7(3H,8H)-dione), RO5126766 (3-[[3-fluoro-2-(methylsulfamoylamino)-4-pyridyl]methyl]-4-methyl-7-pyrimidin-2-yloxy chromen-2-one) or XL-518 ([3,4-difluoro-2-[(2-fluoro-4-iodophenyl)amino]phenyl][3-hydroxy-3-[(2S)-2-piperidinyl] -1-azetidinyl]methanone), although not being limited thereto.

The TGF-β/activin/nodal signaling inhibitor of the present disclosure is specifically E-616452 (2-[3-(6-methyl-2-pyridinyl)-1H-pyrazol-4-yl]-1,5-naphthyridine), A-83-01 (3-(6-methyl-2-pyridinyl)-N-phenyl-4-(4-quinolinyl)-1H-pyrazole-1-carbothioamide) or SB431542 (4-[4-(1,3-benzodioxol-5-yl)-5-(2-pyridinyl)-1H-imidazol-2-yl]benzamide), although not being limited thereto.

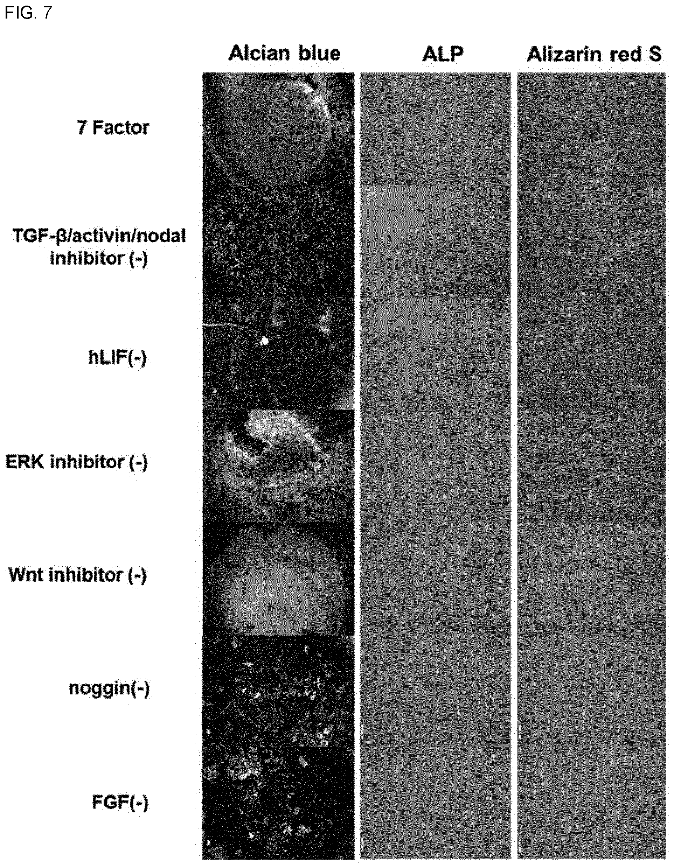

In an example of the present disclosure, when differentiation capacity was compared for the cases where one of the ingredients of the medium, noggin, leukemia inhibitory factor (LIF), basic fibroblast growth factor (bFGF), Wnt signaling activator, extracellular signal-regulated kinase (ERK) signaling inhibitor and TGF-β/activin/nodal signaling inhibitor, was missing with the case where all the ingredients were contained, differentiation into cartilage (alcian blue) or bone (ALP and alizarin red S) was not achieved properly when one of the ingredients was missing (FIG. 7, Table 3).

In addition, when differentiation capacity was compared after replacing noggin with conditioned medium (culture supernatant obtained after culturing CF1 mouse embryonic fibroblasts for 24 hours using complete medium wherein DMEM/F12 is replaced with knockout DMEM (knockout DMEM supplemented with 20% knockout serum replacement (Invitrogen, USA), 1 mM glutamine, 1% nonessential amino acids (Invitrogen, USA), 0.1 mM β-mercaptoethanol, 0.1% penicillin-streptomycin and 5 mg/mL bovine serum albumin), the medium composition of the present disclosure using noggin increased osteogenic differentiation 10 times or more as compared to the conditioned medium and the differentiation period was faster by 1-2 weeks (Tables 1 and 2).

In another aspect of the present disclosure, the present disclosure provides a method for preparing musculoskeletal stem cells, which includes a step of culturing embryonic stem cell (ESC) or induced pluripotent stem cell (iPS) in the medium composition for inducing differentiation into musculoskeletal stem cells.

The culturing may be performed for 5 passages or longer, specifically for 5-25 passages, more specifically for 7-18 passages, without change in the composition of the medium.

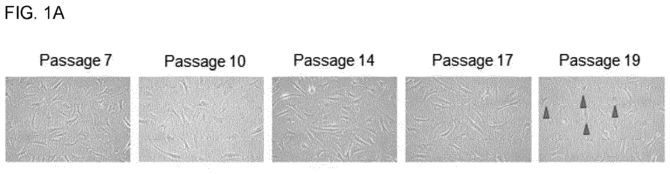

In an example of the present disclosure, the musculoskeletal stem cells differentiated by culturing according to the above method had the same characteristics stably as the cells obtained by subculturing human embryonic stem cells or human induced pluripotent stem cells for 7 passages or longer using a musculoskeletal stem cell induction medium. They grew with similar morphologies for 10 passages or longer, from passage 7 until passage 17, and showed positive result at passage 19 or later when stained with the senescence marker β-galactosidase, indicating the progress of aging (FIG. 1A).

In another aspect of the present disclosure, the present disclosure provides musculoskeletal stem cells prepared using the medium composition for inducing differentiation into musculoskeletal stem cells.

In another aspect of the present disclosure, the present disclosure provides musculoskeletal stem cells (MSSCs) differentiated from embryonic stem cells (ESCs) or induced pluripotent stem cells (iPSCs).

In a specific exemplary embodiment of the present disclosure, the musculoskeletal stem cells of the present disclosure have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In a specific exemplary embodiment of the present disclosure, the musculoskeletal stem cells of the present disclosure further have the following characteristic:
negative for the mesenchymal stem cell marker CD90.

In a specific exemplary embodiment of the present disclosure, the musculoskeletal stem cells of the present disclosure further have the following characteristic:
negative for the mesenchymal stem cell marker CD271.

In a specific exemplary embodiment of the present disclosure, the musculoskeletal stem cells of the present disclosure further have the following characteristic:
positive for the pluripotency marker DPPA4.

In a specific exemplary embodiment of the present disclosure, the musculoskeletal stem cells of the present disclosure further have the following characteristic:
negative for the mesodermal markers T and nodal.

In a specific exemplary embodiment of the present disclosure, the musculoskeletal stem cells of the present disclosure further have the following characteristic:
positive for the neuroectodermal marker Pax6.

In a specific exemplary embodiment of the present disclosure, the musculoskeletal stem cells of the present disclosure further have the following characteristic:
positive for the intestinal stem cell marker LGR5.

In a specific exemplary embodiment of the present disclosure, the musculoskeletal stem cells of the present disclosure further have the following characteristic:
negative for the chondrocyte marker SOX9.

In a specific exemplary embodiment of the present disclosure, the musculoskeletal stem cells of the present disclosure further have the following characteristic:
negative for the myoblast marker MyoD.

In a specific exemplary embodiment of the present disclosure, the musculoskeletal stem cells of the present disclosure are positive for CD10, CD44, CD105, CD146 and/or CD166.

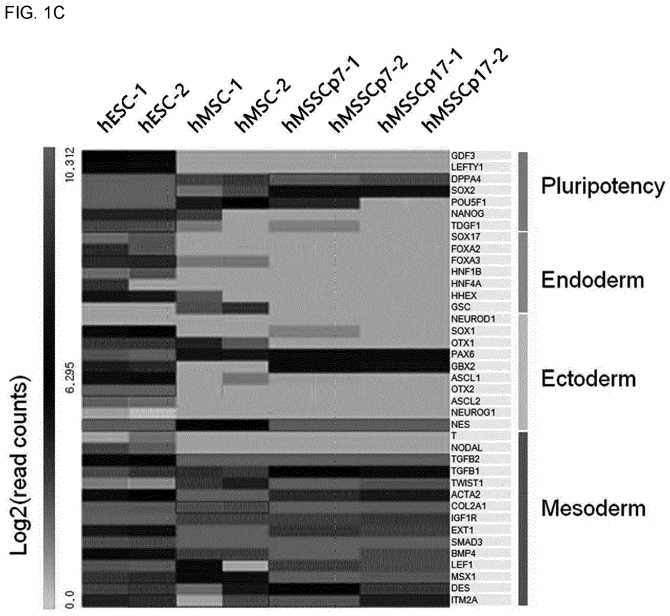

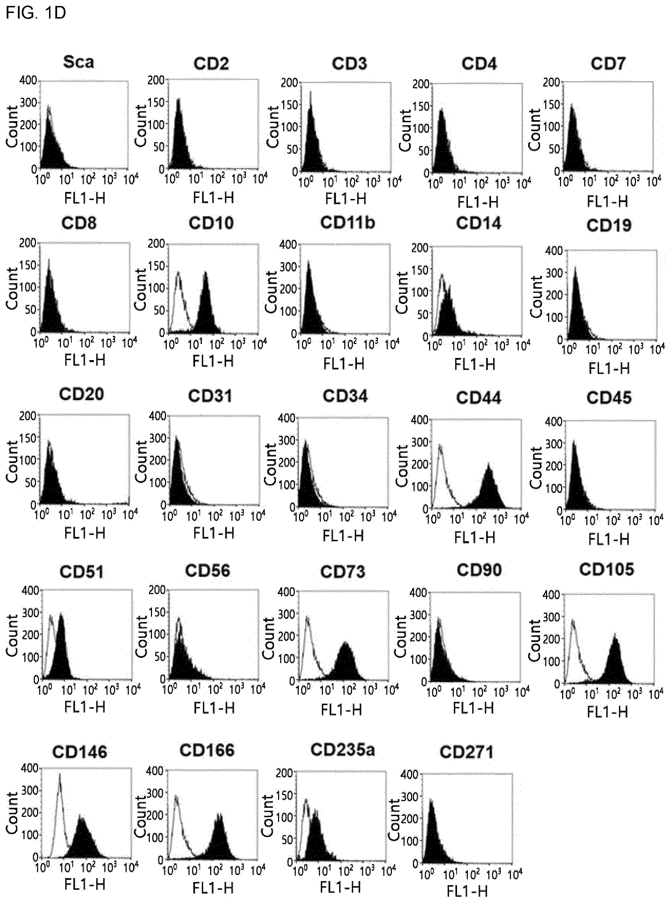

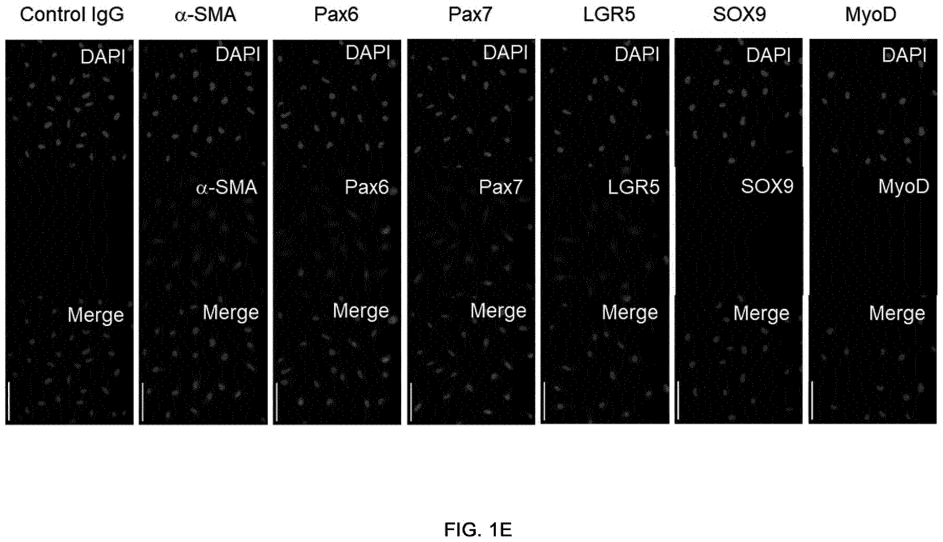

In an example of the present disclosure, the musculoskeletal stem cells of the present disclosure did not show the expression of most pluripotency markers but showed the expression of DPPA4, and they were positive for the ectodermal marker NES. In addition, they were positive for most mesodermal markers excluding DES and early mesodermal markers T and nodal, and were negative for most endodermal markers (FIG. 1C). In addition, as a result of investigating the expression of mesenchymal stem cell-specific cell surface antigens for characterization of hMSSCs, the mesenchymal stem cell markers CD44, CD51, CD73, CD105, CD146 and CD166 were expressed in hMSSCs, whereas the mesenchymal stem cell markers CD90 and CD271 were not expressed in hMSSCs. In addition, whereas the blood-associated cell surface markers CD2, CD3, CD7, CD8, CD11b, CD14, CD19, CD20, CD31, CD34 and CD56 were not expressed, the pre-B cell marker CD10 was expressed (FIG. 1D). Additionally, when the expression of tissue-specific markers of different lineages was investigated, the mesodermal marker alpha smooth muscle actin (a-SMA), the neuroectodermal marker Pax6, the myogenic satellite marker Pax7, the intestinal stem cell marker LGR5, etc. were expressed, and the chondrocyte marker SOX9, the myoblast marker MyoD, etc. were not expressed (FIG. 1E).

In a specific exemplary embodiment of the present disclosure, the musculoskeletal stem cells differentiate into the mesoderm, but not into the ectoderm or endoderm.

In a specific exemplary embodiment of the present disclosure, the musculoskeletal stem cells differentiate into muscle, bone, cartilage, tendon or ligament.

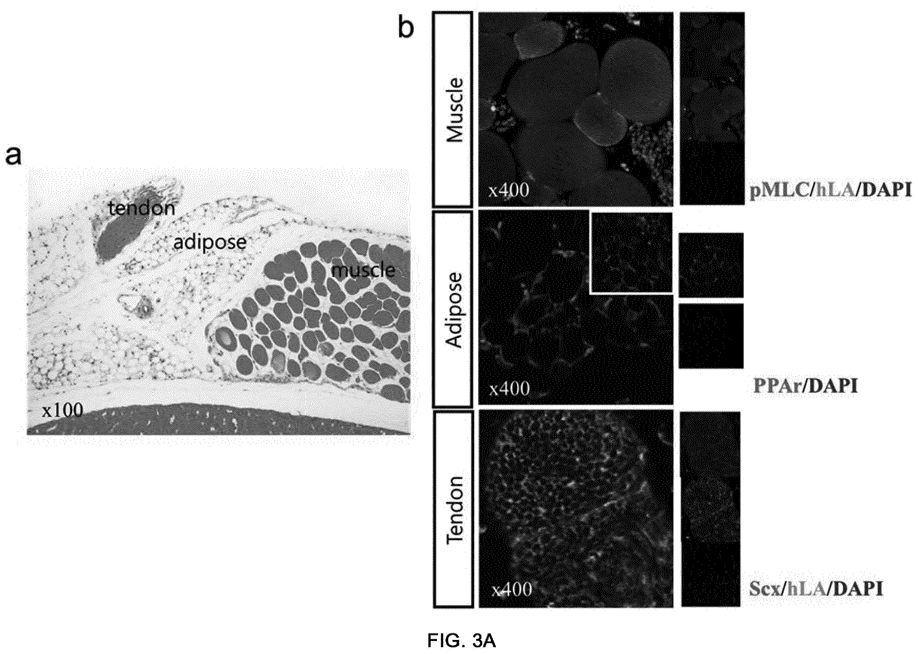

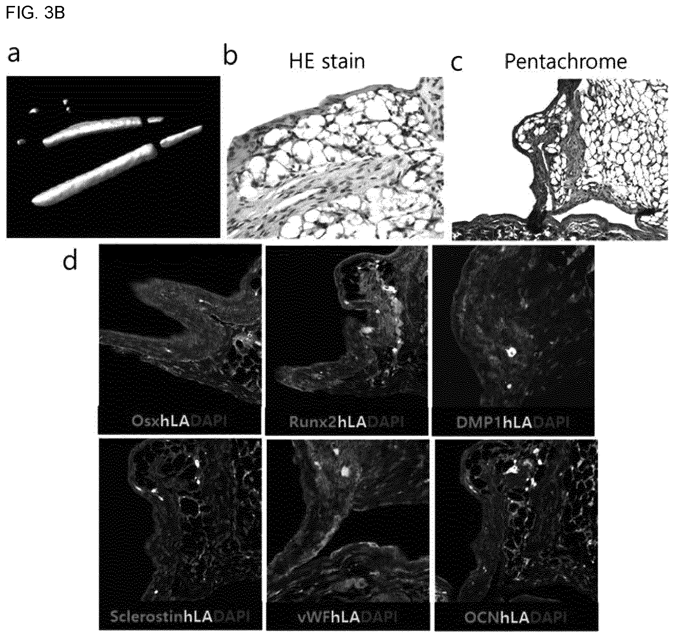

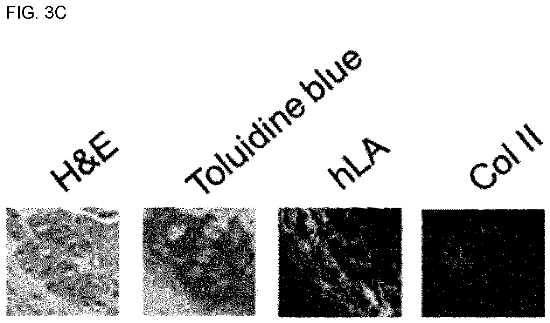

In an example of the present disclosure, when the musculoskeletal stem cells of the present disclosure were cultured in a mesenchymal stem cell culturing medium (e.g., MSCGM, MSCGM-CD, etc.) and then transplanted in the kidney (kidney capsule) or subcutaneous tissue, the typical formation of muscle, adipose, tendon, bone and cartilage in the kidney or subcutaneous tissue was observed (FIG. 3). As a result of investigating the differentiated muscle tissues, it was confirmed that all the cells were differentiated into skeletal muscle, not into smooth muscle. Unlike the result of in-vitro experiment where differentiation into adipose did not occur, they could differentiate into adipose in in-vivo experiment.

In a specific exemplary embodiment of the present disclosure, the musculoskeletal stem cells do not differentiate into nerve.

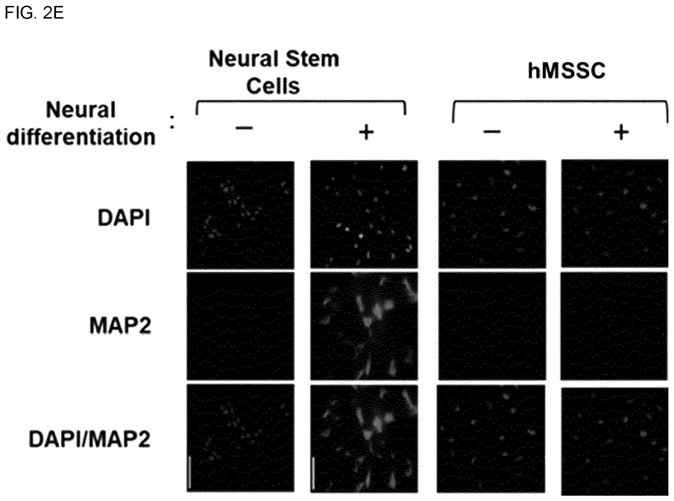

In an example of the present disclosure, as a result of differentiating the musculoskeletal stem cells into nerve cells in a neural differentiation medium and investigating using a nerve cell marker, it was confirmed that the musculoskeletal stem cells have no potential for differentiation into nerve cells (FIG. 2E).

In a specific exemplary embodiment of the present disclosure, the musculoskeletal stem cells do not differentiate into endothelial cells.

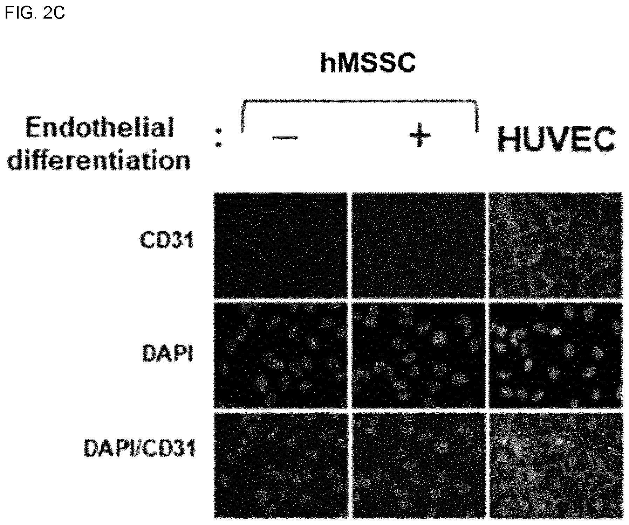

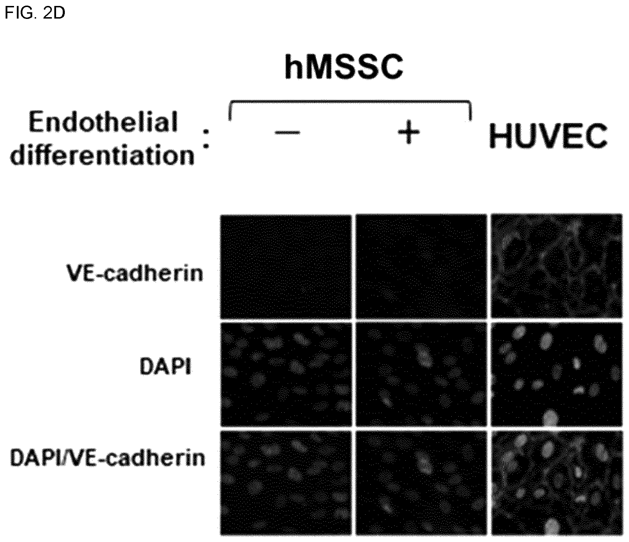

In an example of the present disclosure, as a result of differentiating the musculoskeletal stem cells into endothelial cells using an endothelial cell (EC) growth medium and investigating using an endothelial cell marker, it was confirmed that the musculoskeletal stem cells have no potential for differentiation into endothelial cells (FIGS. 2C and 2D).

The musculoskeletal stem cells were deposited on October 10, 2018 in the Korean Cell Line Bank with the accession number KCLRF-BP-00460.

In another aspect of the present disclosure, the present disclosure provides a pharmaceutical composition for preventing or treating a musculoskeletal disease, which contains the musculoskeletal stem cells.

In another aspect of the present disclosure, the present disclosure provides a cell therapeutic agent containing the musculoskeletal stem cells.

In another aspect of the present disclosure, the present disclosure provides a use of a pharmaceutical composition for preventing or treating a musculoskeletal disease, which contains the musculoskeletal stem cells.

In another aspect of the present disclosure, the present disclosure provides a method for preventing or treating a musculoskeletal disease, which includes a step of administering the musculoskeletal stem cells to a patient.

In the present specification, the term "musculoskeletal disease" refers to a disease occurring in bone, cartilage, tooth, muscle, ligament, tendon, nerve, etc. and is specifically one or more disease selected from a group consisting of osteoporosis, osteomalacia, osteogenesis imperfecta, osteopetrosis, osteosclerosis, Paget's disease, bone cancer, arthritis, rheumatoid arthritis, rickets, fracture, periodontal disease, tooth decay, gingivitis, periodontitis, alveolar bone loss, tooth damage or loss, segmental bone defect, osteolytic bone disease, primary and secondary hyperparathyroidism, hyperostosis, degenerative arthritis, deformative gonarthrosis, deformative coxarthrosis, deformative foot arthrosis, deformative hand arthrosis, deformative shoulder arthrosis, deformative elbow arthrosis, chondromalacia patella, simple knee arthritis, osteochondritis dissecans, lateral epicondylitis, medial epicondylitis, Heberden's nodes, Bouchard's nodes, deformative thumb CM arthrosis, meniscus damage, intervertebral disc degeneration, cruciate ligament biceps tendon injury, cruciate ligament tear, ligament damage, tendon injury, frozen shoulder, rheumatic fever, diffuse systemic sclerosis, systemic lupus erythematosus, Ehlers-Danlos syndrome, Marfan's syndrome, scurvy, quadriceps tear, knee joint ligament tear, peroneal tendon disease, Achilles tendon rupture, plantar fasciitis, rotator cuff tear, calcific tendinitis, shoulder impingement syndrome, recurrent dislocation, habitual dislocation, sarcopenia, myasthenia gravis, myotonia, amyotrophic lateral sclerosis, Lou Gehrig's disease, inflammatory myopathy, Charcot-Marie-Tooth disease and muscular dystrophy, although not being limited thereto.

The pharmaceutical composition of the present disclosure may contain a pharmaceutically acceptable carrier. The pharmaceutically acceptable carrier contained in the composition is one commonly used in preparation and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. The pharmaceutical composition may further contain a lubricant, a wetting agent, a sweetener, a flavorant, an emulsifier, a suspending agent, a preservative, etc. in addition to the above-described ingredients.

The pharmaceutical composition of the present disclosure may be administered orally or parenterally. For parenteral administration, it may be administered via intravenous injection, subcutaneous injection, intramuscular injection, intraarticular injection, intraosseous injection, injection into the tendon or ligament, injection into the gum, intraperitoneal injection, endothelial administration, topical administration, intranasal administration, intrapulmonary administration, intrarectal administration, etc. In addition, the composition may be administered by any device allowing the active ingredient to be delivered to a target cell.

An adequate administration dosage of the pharmaceutical composition of the present disclosure varies depending on various factors such as formulation method, administration method, the age, body weight, sex, pathological condition and diet of a patient, administration time, administration route, excretion rate and response sensitivity. A preferable administration dose of the composition is 10²-10¹⁰ cells/kg for an adult. The term pharmaceutically effective amount refers to an amount sufficient to prevent or treat a musculoskeletal disease.

The composition of the present disclosure may be prepared into a unit-dose form by formulating using a pharmaceutically acceptable carrier and/or excipient or may be introduced into a multi-dose container according to a method that can be easily executed by those having ordinary knowledge in the art to which the present disclosure belongs. The formulation may be in the form of a solution in an oil or an aqueous medium, a suspension, an emulsion, an extract, a powder, a granule, a tablet or a capsule, and may further contain a dispersant or a stabilizer. In addition, the composition may be administered as an individual therapeutic agent or in combination with another therapeutic agent. In case of co-administration, it may be administered sequentially or simultaneously with the existing therapeutic agent. Also, it may be administered once or multiple times, if necessary.

In the present disclosure, the term "cell therapeutic agent" refers to cells or tissues isolated from human and prepared as a medication through culturing and special operation for use in treatment, diagnosis and prevention (USFDA definition). It is used for the purpose of treatment, diagnosis and prevention by proliferating and screening living autologous, homologous or heterologous cells ex vivo or otherwise changing the biological properties of the cells to restore the functions of cells or tissues.

In the present disclosure, the term "prevention" refers to any action of suppressing a musculoskeletal disease or delaying the progress thereof by administering the composition or cell therapeutic agent of the present disclosure.

The term "treatment" used in the present disclosure refers to any action of improving or beneficially changing a musculoskeletal disease by administering the composition or cell therapeutic agent of the present disclosure.

The pharmaceutical composition or cell therapeutic agent of the present disclosure may be used for human or an animal.

The pharmaceutical composition or cell therapeutic agent of the present disclosure may be used either alone or in combination with surgery, radiation therapy, hormone therapy, chemotherapy, a biological response modifier, implantation of artificial joint, artificial cartilage, etc., regenerative therapy, etc. for prevention and treatment of musculoskeletal disease.

In another aspect of the present disclosure, the present disclosure provides a method for screening musculoskeletal stem cells.

In a specific exemplary embodiment of the present disclosure, the present disclosure includes a step of selecting cells having the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In a specific exemplary embodiment of the present disclosure, the present disclosure further includes a step of selecting cells having the following characteristic:
negative for the mesenchymal stem cell marker CD90.

In a specific exemplary embodiment of the present disclosure, the present disclosure further includes a step of selecting cells having the following characteristic:
negative for the mesenchymal stem cell marker CD271.

In a specific exemplary embodiment of the present disclosure, the present disclosure further includes a step of selecting cells having the following characteristic:
positive for the pluripotency marker DPPA4.

In a specific exemplary embodiment of the present disclosure, the present disclosure further includes a step of selecting cells having the following characteristic:
negative for the mesodermal markers T and nodal.

In a specific exemplary embodiment of the present disclosure, the present disclosure further includes a step of selecting cells having the following characteristic:
positive for the neuroectodermal marker Pax6.

In a specific exemplary embodiment of the present disclosure, the present disclosure further includes a step of selecting cells having the following characteristic:
positive for the intestinal stem cell marker LGR5.

In a specific exemplary embodiment of the present disclosure, the present disclosure further includes a step of selecting cells having the following characteristic:
negative for the chondrocyte marker SOX9.

In a specific exemplary embodiment of the present disclosure, the present disclosure further includes a step of selecting cells having the following characteristic:
negative for the myoblast marker MyoD.

In a specific exemplary embodiment of the present disclosure, the present disclosure further includes a step of selecting cells positive for CD10, CD44, CD105, CD146 and/or CD166.

Musculoskeletal stem cells that can effectively differentiate into bone, tooth, cartilage, tendon, ligament, muscle, etc. can be screened easily by using the screening method of the present disclosure.

In another aspect of the present disclosure, the present disclosure provides a medium composition for selectively inducing differentiation into only muscle from musculoskeletal stem cells (MSSCs) that can be differentiated into bone, tooth, cartilage, tendon, ligament, muscle and adipose.

In a specific exemplary embodiment of the present disclosure, the medium composition of the present disclosure contains heparin.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In another aspect of the present disclosure, the present disclosure provides a pharmaceutical composition for preventing or treating a musculoskeletal disease, which contains the medium composition for inducing differentiation and musculoskeletal stem cells as active ingredients.

In another aspect of the present disclosure, the present disclosure provides a method for inducing selective differentiation from musculoskeletal stem cells (MSSCs) to only muscle, including a step of treating heparin in musculoskeletal stem cells that can be differentiated into bone, tooth, cartilage, tendon, ligament, muscle and adipose.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

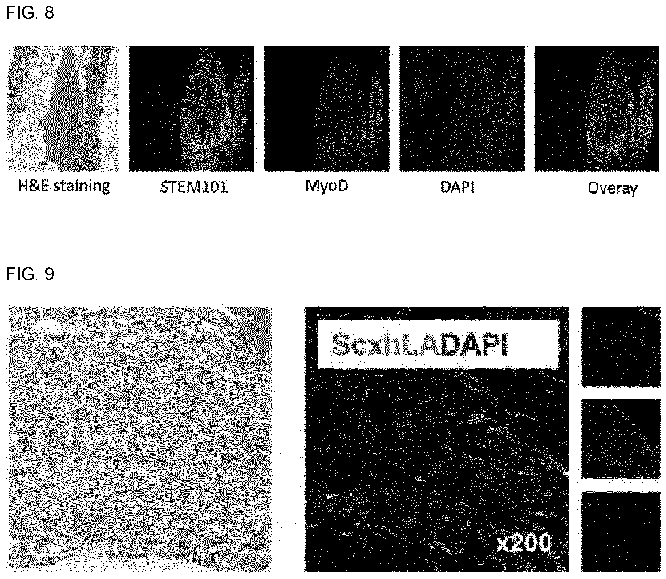

In an example of the present disclosure, it was confirmed that the musculoskeletal stem cells of the present disclosure were differentiated selectively into muscle only when they were transplanted into the subcutaneous tissue of mouse after being mixed with heparin (FIG. 8).

In another aspect of the present disclosure, the present disclosure provides a medium composition for selectively inducing differentiation into only tendon or ligament from from musculoskeletal stem cells (MSSCs) that can be differentiated into bone, tooth, cartilage, tendon, ligament, muscle and adipose.

In a specific exemplary embodiment of the present disclosure, the medium composition of the present disclosure contains connective tissue growth factor (CTGF) and ascorbic acid.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In another aspect of the present disclosure, the present disclosure provides a pharmaceutical composition for preventing or treating a musculoskeletal disease, which contains musculoskeletal stem cells pretreated with the medium composition for inducing differentiation as active ingredients.

In another aspect of the present disclosure, the present disclosure provides a method for inducing selective differentiation from musculoskeletal stem cells (MSSCs) to only tendon or ligament, including a step of treating connective tissue growth factor (CTGF) and ascorbic acid in musculoskeletal stem cells that can be differentiated into bone, tooth, cartilage, tendon, ligament, muscle and adipose.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In an example of the present disclosure, it was confirmed that the musculoskeletal stem cells of the present disclosure were differentiated selectively into tendon or ligament only when they were transplanted into the subcutaneous tissue of mouse after being pretreated with CTGF and ascorbic acid (FIG. 9).

The mixing ratio of the musculoskeletal stem cells with CTGF and ascorbic acid is not limited. Specifically, 1×10⁵-1×10⁷ musculoskeletal stem cells may be pretreated for 1-7 days with 5-500 ng/mL CTGF and 2.5-250 µg/mL ascorbic acid.

In another aspect of the present disclosure, the present disclosure provides a medium composition for selectively inducing differentiation into only bone from musculoskeletal stem cells (MSSCs) that can be differentiated into bone, tooth, cartilage, tendon, ligament, muscle and adipose.

In a specific exemplary embodiment of the present disclosure, the medium composition of the present disclosure contains insulin, hyaluronic acid and ascorbic acid.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In another aspect of the present disclosure, the present disclosure provides a pharmaceutical composition for preventing or treating a musculoskeletal disease, which contains musculoskeletal stem cells pretreated with the medium composition for inducing differentiation as active ingredients.

In another aspect of the present disclosure, the present disclosure provides a method for inducing selective differentiation from musculoskeletal stem cells to only bone, including a step of treating insulin, hyaluronic acid and ascorbic acid in that can be differentiated into bone, tooth, cartilage, tendon, ligament, muscle and adipose.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

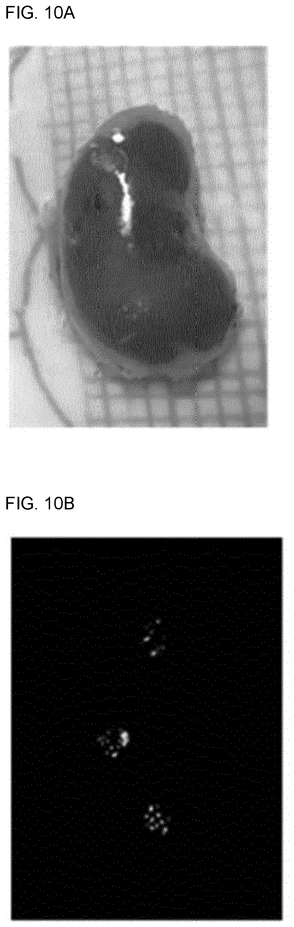

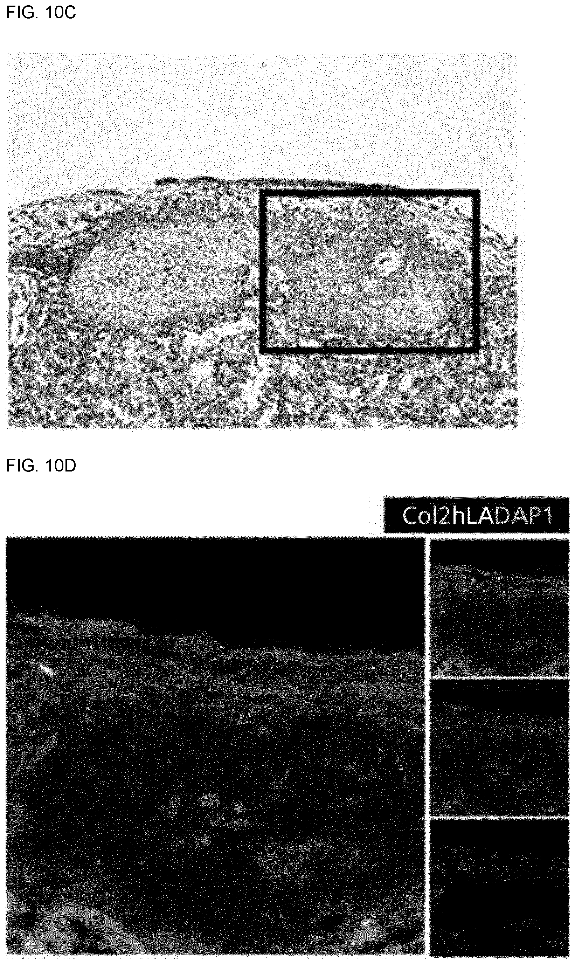

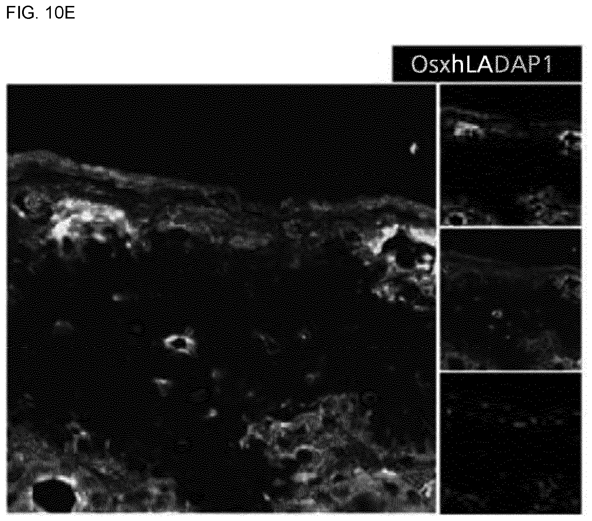

In an example of the present disclosure, it was confirmed that the musculoskeletal stem cells of the present disclosure were differentiated selectively into bone only when they were transplanted into the kidney of mouse after being pretreated with insulin, ascorbic acid and hyaluronic acid (FIG. 10).

The mixing ratio of the musculoskeletal stem cells with insulin, ascorbic acid and hyaluronic acid is not limited. Specifically, 1×10⁵-1×10⁷ musculoskeletal stem cells may be pretreated for 1-7 days with 0.5-50 µg/mL insulin, 2.5-250 µg/mL ascorbic acid and 0.1-10 µg/mL hyaluronic acid.

In another aspect of the present disclosure, the present disclosure provides a medium composition for selectively inducing differentiation into only tooth from musculoskeletal stem cells (MSSCs) that can be differentiated into bone, tooth, cartilage, tendon, ligament, muscle and adipose.

In a specific exemplary embodiment of the present disclosure, the medium composition of the present disclosure contains dental epithelial cells.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

In another aspect of the present disclosure, the present disclosure provides a pharmaceutical composition for preventing or treating a musculoskeletal disease, which contains musculoskeletal stem cells pretreated with the medium composition for inducing differentiation as active ingredients.

In another aspect of the present disclosure, the present disclosure provides a method for inducing selective differentiation from musculoskeletal stem cells to only tooth, including a step of coating dental epithelial cells on musculoskeletal stem cells that can be differentiated into bone, tooth, cartilage, tendon, ligament, muscle and adipose..

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

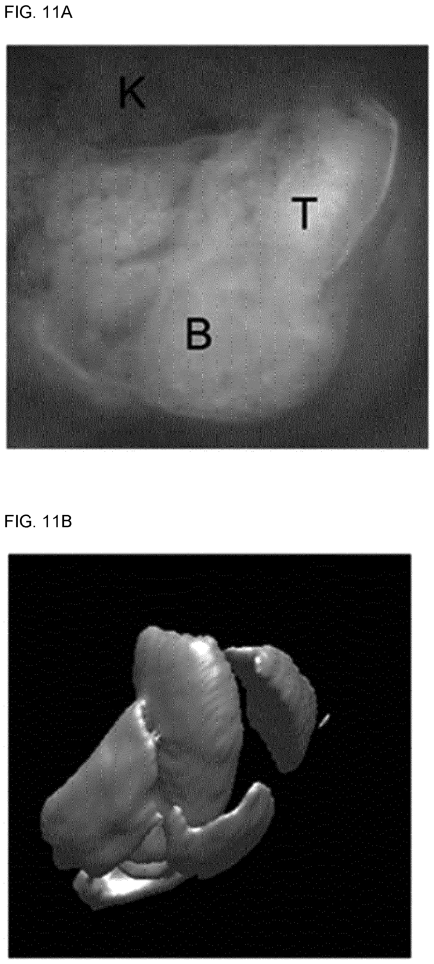

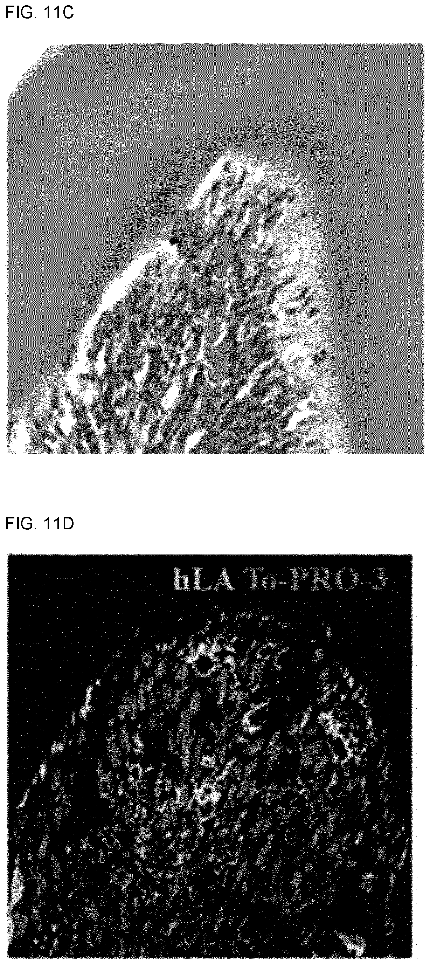

In an example of the present disclosure, it was confirmed that the musculoskeletal stem cells of the present disclosure were differentiated selectively into tooth only when they were transplanted into the kidney of mouse after being coated with dental epithelial cells (FIG. 11).

In another aspect of the present disclosure, the present disclosure provides a method for treating damaged tendon or ligament, which includes a step of administering musculoskeletal stem cells (MSSCs) that can differentiate into bone, tooth, cartilage, tendon, ligament, muscle and adipose to a damaged tendon or ligament area of a subject.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

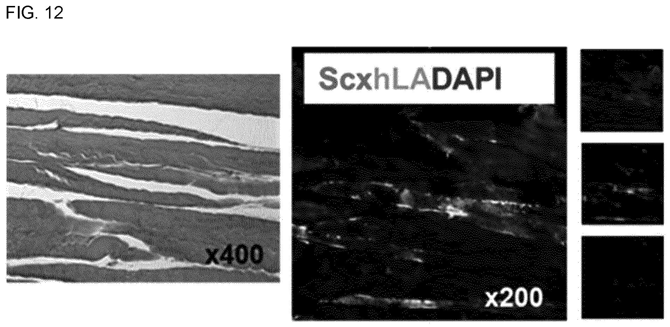

The musculoskeletal stem cells may be injected directly to the damaged tendon or ligament area or may be mixed with an adhesive such as fibrin glue, cyanoacrylate, gelatin glue, polyurethane, etc. in order to improve adhesion to the tendon or ligament area. For example, a cell aggregate may be prepared by mixing 1×10⁵-1×10⁷ musculoskeletal stem cells with 1-100 µL of fibrin glue and then transplanted (FIG. 12).

In another aspect of the present disclosure, the present disclosure provides a method for treating arthritis, which includes a step of administering musculoskeletal stem cells (MSSCs) that can differentiate into bone, tooth, cartilage, tendon, ligament, muscle and adipose into the articular cavity of a subject.

In a specific exemplary embodiment of the present disclosure, the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

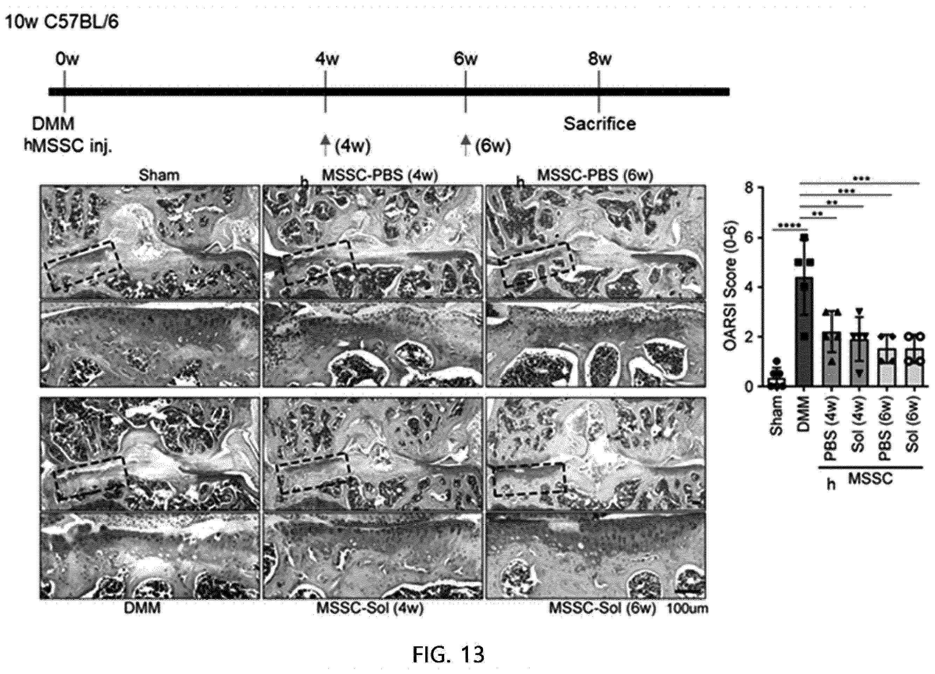

The musculoskeletal stem cells may be injected by various methods, including direct injection to a damaged cartilage area (e.g., articular cavity), injection after resuspension in PBS, injection after freeze-drying and thawing, etc. (FIG. 13).

The musculoskeletal stem cells described in the present disclosure may further have the following characteristics:
e) negative for the mesenchymal stem cell marker CD90;
f) negative for the mesenchymal stem cell marker CD271;
g) positive for the pluripotency marker DPPA4;
h) negative for the mesodermal markers T and nodal;
i) positive for the neuroectodermal marker Pax6;
j) positive for the intestinal stem cell marker LGR5;
k) negative for the chondrocyte marker SOX9; or
l) negative for the myoblast marker MyoD.

### [Advantageous Effects]

The features and advantages of the present disclosure are summarized as follows:
(i) The present disclosure provides musculoskeletal stem cells derived from ESCs or iPSCs.
(ii) The present disclosure also provides a medium composition for inducing differentiation, which induces differentiation of the musculoskeletal stem cells into muscle, cartilage, tendon, ligament, bone or tooth, and a method for inducing the differentiation.
(iii) The musculoskeletal stem cells of the present disclosure may be usefully used for prevention or treatment of various musculoskeletal diseases because they can be differentiated into a desired tissue only with directionality through simple operation.

### [Brief Description of Drawings]

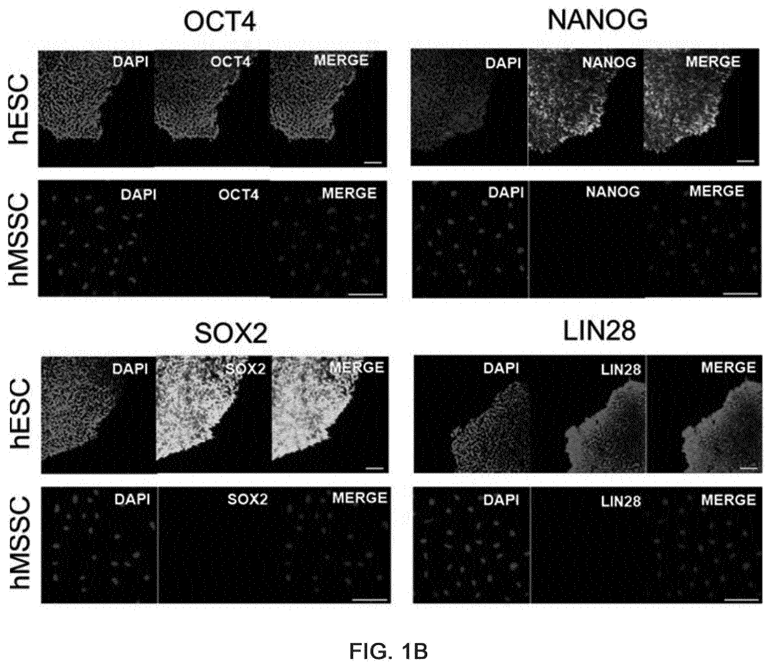

FIGS. 1A to 1E show the characteristics of hMSSCs derived from hESCs. FIG. 1A shows the change of cell morphology of hESCs subcultured using a musculoskeletal stem cell induction medium, from passage 7 to passage 19. FIG. 1B shows a result of observing the expression of the pluripotency markers OCT4, NANOG, SOX2 and LIN28 in hMSSCs by immunocytochemistry. FIG. 1C shows a result of investigating the expression of pluripotency, ectodermal, mesodermal and endodermal markers in hESCs, hMSCs and hMSSCs at passages 7 and 17 twice through RNA sequencing. FIG. 1D shows a result of measuring the expression of cell surface antigens by flow cytometry for characterization of hMSSCs. FIG. 1E shows a result of measuring the expression of tissue-specific markers of different lineages by immunocytochemistry for characterization of hMSSCs. In the figures, DAPI indicates stained nuclei. In the figures, blue triangles indicate β-galactosidase-positive cells.

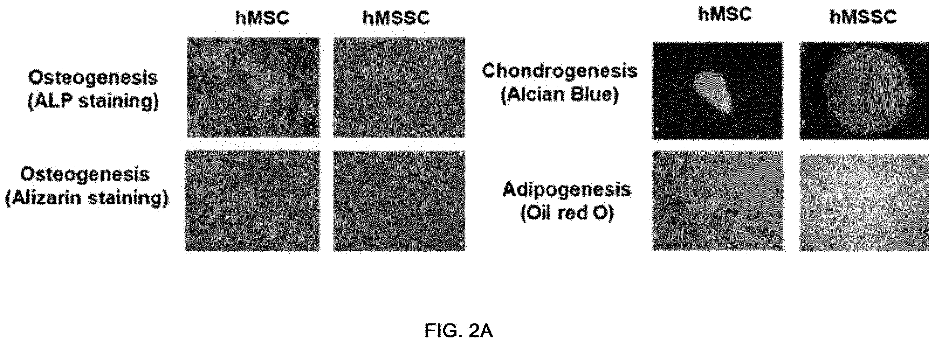

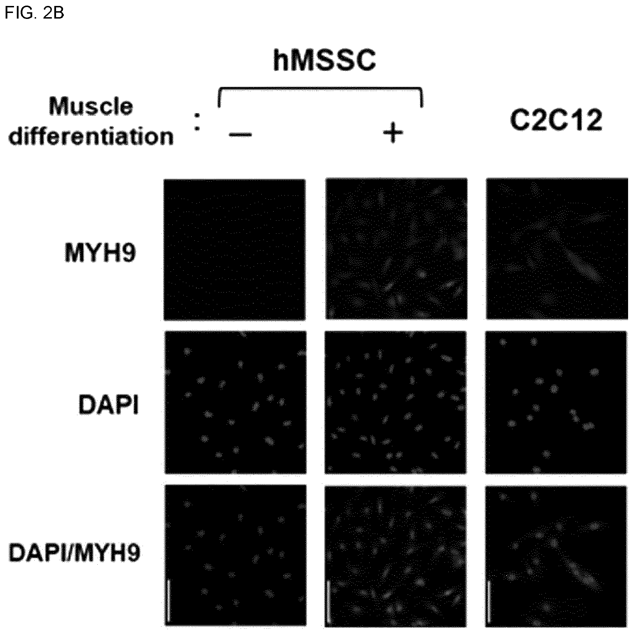

FIGS. 2A to 2E show a result of comparing the in-vitro differentiation capacity of hMSSCs with other types of cells. FIG. 2A shows a result of comparing the in-vitro bone, cartilage and adipose differentiation capacity of hMSCs and hMSSCs. FIG. 2B shows a result of confirming the potential of hMSSCs for differentiation into skeletal muscle by immunocytochemistry for the skeletal muscle cell-specific marker MYH9. C2C12 cells were used as a skeletal muscle cell positive control group. FIGS. 2C and 2D show a result of confirming the absence of the potential of hMSSCs for differentiation into endothelial cells by immunocytochemistry for the endothelial cell-specific markers CD31 and VE-cadherin. FIG. 2E show a result of confirming the absence of the potential of hMSSCs for differentiation into nerve cells by immunocytochemistry for the nerve cell-specific marker MAP2. Neural stem cells derived from H9 hESCs were used as a positive control group.
FIGS. 3A to 3C show a result of measuring the differentiation potential of hMSSCs in vivo. FIG. 3A-a shows a result of confirming the formation of typical muscle, adipose and tendon by H&E staining when hMSSCs were transplanted into the kidney. FIG. 3A-b shows a result of confirming the differentiation into muscle, adipose and tendon cells when hMSSCs were transplanted into the kidney with immunohistochemistry for the muscle-specific marker pMLC, the adipose-specific marker PPAR-gamma (PPAr), or the tendon- or ligament-specific marker SCX. hLA shows a result of staining of a human cell-specific marker for confirming the presence of human-derived cells. FIG. 3B-a shows a result of confirming the formation of bone in the kidney where hMSSCs were transplanted by micro-CT scan. FIGS. 3B-b and 3B-c show a result of confirming the formation of bone by H&E and pentachrome immunohistochemistry. FIG. 3B-d shows a result of confirming the expression of the human cell marker hLA (human leukocyte antigen), the bone markers Osx (osterix), Runx2, DMP1 and OCN (osteocalin), and the blood vessel marker vWF in osteogenic tissue by immunohistochemistry. FIG. 3C shows a result of confirming cartilage formation when hMSSCs were transplanted into the subcutaneous tissue by H&E and toluidine blue immunohistochemistry for chondrocytes. In addition, the expression of the cartilage marker Colli (collagen type II) was confirmed by immunohistochemistry.

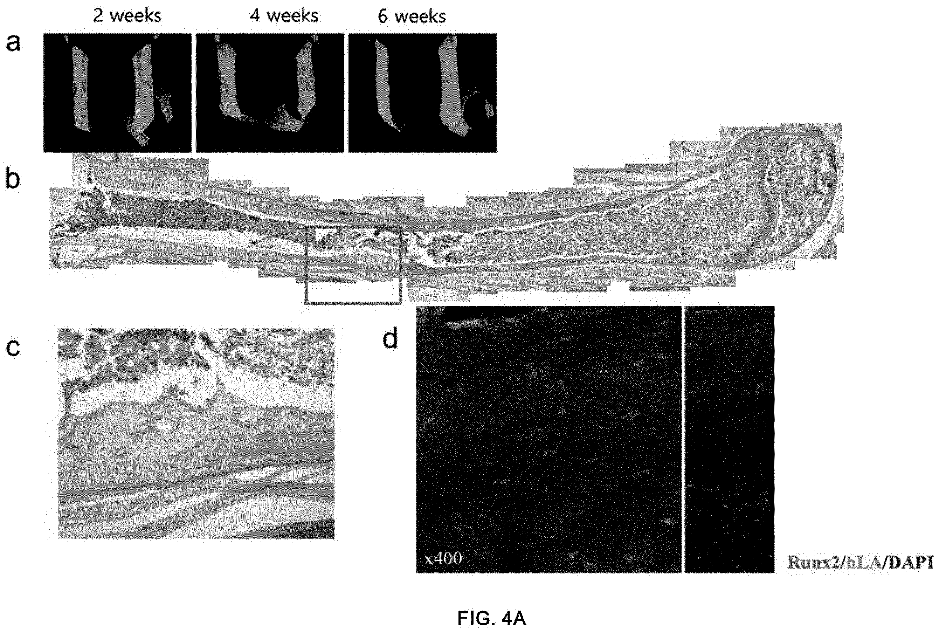

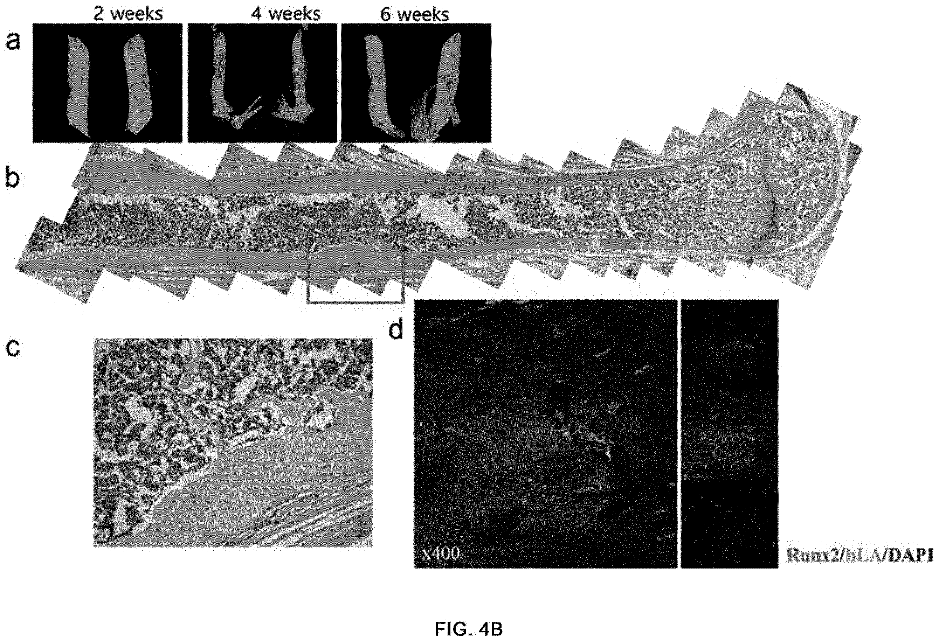

FIGS. 4A and 4B show a result of confirming the effect of hMSSCs on recovery from fracture. FIG. 4A shows that bone was formed by the cells of mouse, not by hMSCs transplanted into the fracture area. FIG. 4A-a shows a result of micro-CT at 2, 4 and 6 weeks after the transplantation of hMSCs into the fracture area. FIG. 4A-b shows a result of H&E immunohistochemistry of the thighbone including the fracture area into which hMSCs were transplanted. FIG. 4A-c is an enlarged image of the red square in FIG. 4A-b. FIG. 4A-d shows a result of confirming that the transplanted hMSCs were not differentiated into bone cells by immunohistochemistry for the bone cell marker Runx2 and the human cell marker hLA. FIG. 4B shows that bone was formed by differentiation of hMSSCs when the hMSCs when the hMSSCs were transplanted, unlike the hMSCs. FIG. 4B-a shows a result of micro-CT at 2, 4 and 6 weeks after the transplantation of hMSSCs into the fracture area. FIG. 4B-b shows a result of H&E immunohistochemistry of the thighbone including the fracture area into which hMSSCs were transplanted. FIG. 4B-c is an enlarged image of the red square in FIG. 4B-b. FIG. 4B-d shows a result of confirming that the transplanted hMSSCs were differentiated into bone cells by immunohistochemistry for the bone cell marker Runx2 and the human cell marker hLA.

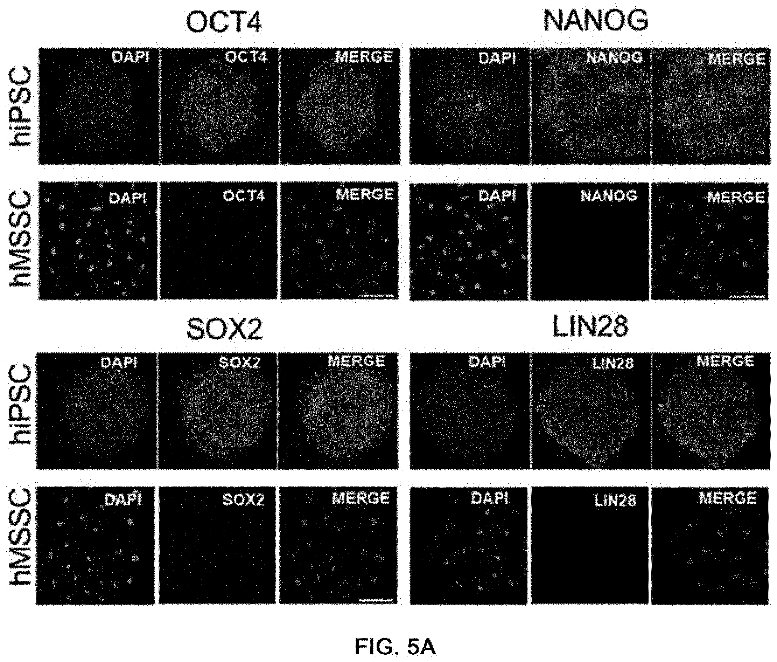

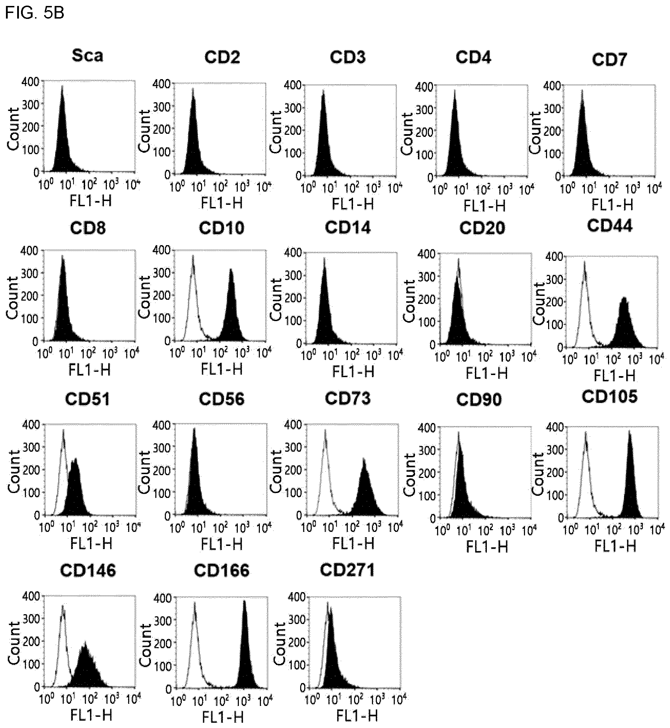

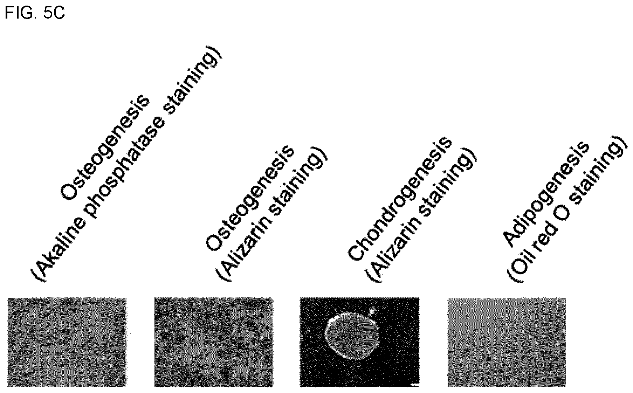

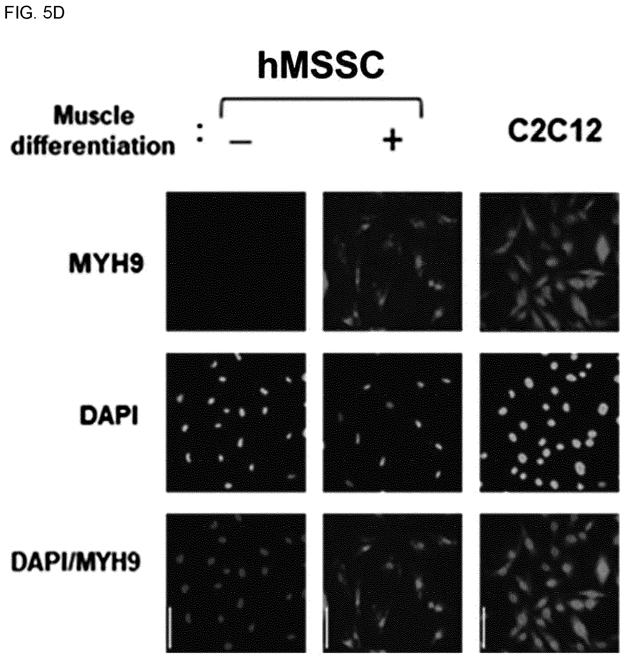

FIGS. 5A to 5D show a result of investigating whether hiPSCs are also differentiated into hMSSCs like hESCs. FIG. 5A shows a result of investigating the expression level of the pluripotency markers Oct4, Nanog, Sox2 and Lin28 in hMSSCs derived from hiPSCs by immunocytochemistry. FIG. 5B shows a result of investigating the expression of specific cell surface antigens in hMSSCs derived from hiPSCs by flow cytometry. FIG. 5C shows a result of confirming the in-vitro bone, cartilage and adipose differentiation capacity of hMSSCs derived from hiPSCs. FIG. 5D shows a result of differentiating hMSSCs derived from hiPSCs into skeletal muscle by culturing using a skeletal muscle differentiation medium and conducting immunocytochemistry for the skeletal muscle marker MYH9.

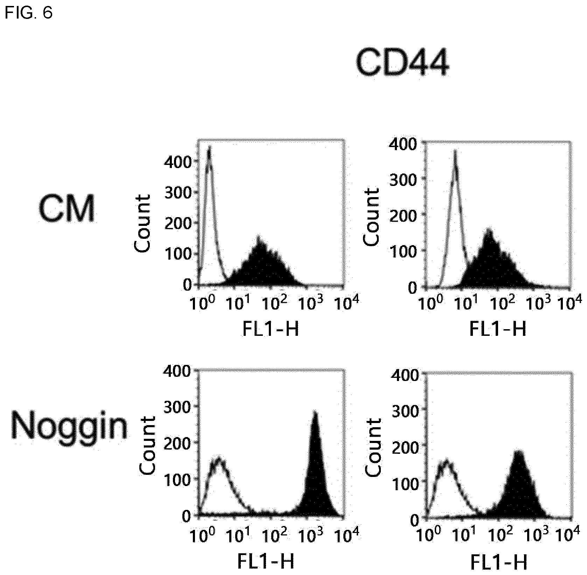

FIG. 6 shows a result of comparing the expression level of CD44 in hMSSCs induced with a CM medium and an hMSSC induction medium by flow cytometry.
FIG. 7 shows a result of investigating the effect of an hMSSC medium ingredients deficiency on differentiation into cartilage or bone by staining with alcian blue which confirms cartilage differentiation and ALP and alizarin red S which confirm osteogenic differentiation.
FIG. 8 shows the selective differentiation of hMSSCs derived from hESCs into muscle. hMSSCs (1×10⁶ cells) differentiated from hESCs were transplanted into the subcutaneous tissue of BALB/c nude mouse after being mixed with fibrin glue. After 7 weeks, the transplanted area was removed and then stained with hematoxylin & eosin.
FIG. 9 shows the selective differentiation of hMSSCs derived from hESCs into tendon or ligament. hMSSCs were pretreated with 50 ng/mL connective tissue growth factor and 25 µg/mL ascorbic acid for 2 days and the pretreated cells (1×10⁶ cells) were transplanted into the subcutaneous tissue of BALB/c nude mouse after being mixed with 100 µL of fibrin glue. After 5 weeks, the transplanted area was removed and then stained with hematoxylin & eosin. It was also stained with an antibody against hLA which binds to a human cell nucleus marker to confirm whether the differentiated tissue is derived from the hMSSCs, and immunofluorescence staining with an antibody against the tendon or ligament marker SCX marker to confirm whether the differentiated tissue is tendon or ligament.
FIG. 10 shows the selective differentiation of hMSSCs derived from hESCs into bone. hMSSCs derived from hESCs were prepared into a cell aggregate by a hanging drop method using N2B27 medium supplemented with 10 µg/mL hyaluronic acid, 5 µg/mL insulin and 25 µg/mL ascorbic acid and transplanted into the kidney of BALB/c nude mouse after culturing for 3 days. 5 weeks later, the transplanted area was removed and then subjected to hematoxylin & eosin staining, micro-CT and immunostaining. FIG. 10A shows the image of the kidney extracted 5 weeks after the transplantation of the hMSSC cell aggregate. FIG. 10B shows the micro-CT image of the kidney of FIG. 10A. FIG. 10C shows the typical bone formation by H&E staining in the hard tissue area on micro-CT image 4 weeks after transplantation of the hMSSC cell aggregates into the kidney. FIG. 10D shows a result of investigating the expression of the human cell marker hLA (human leukocyte antigen) and the cartilage marker Colli (collagen type II) in cells in osteogenic tissue by immunohistochemistry. FIG. 10E shows a result of investigating the expression of the human cell marker hLA (human leukocyte antigen) and the bone marker Osx (osterix) in cells of osteogenic tissue by immunohistochemistry.
FIG. 11 shows the selective differentiation of hMSSCs derived from hESCs into tooth. FIG. 11A shows the image of the kidney extracted 6 weeks after the transplantation of an hMSSC cell aggregate into the kidney of mouse by coating with mouse dental epithelial cell. K indicates kidney, B indicates bone, and T indicates tooth. FIG. 11B shows the micro-CT image of the kidney of FIG. 11A. FIG. 11C shows a hematoxylin & eosin staining image of the tooth of FIG. 11A. FIG. 11D shows a result of investigating the expression of the human cell marker hLA for the tooth of FIG. 11A by immunohistochemistry. To-PRO-3 shows a result of staining nuclei.
FIG. 12 shows a result of investigating the possibility of treatment of damaged ligament with hMSSCs derived from hESCs in vivo. It was confirmed by H&E staining and immunofluorescence staining for the human cell-specific marker hLA and the tendon- or ligament-specific marker SCX that broken ligament was connected as the hMSSCs were differentiated..
FIG. 13 shows a result of investigating the possibility of treatment of damaged cartilage with hMSSCs derived from hESCs in vivo. It was confirmed that the severity of arthritis was decreased to half or lower in the groups to which hMSSCs were injected (PBS and Sol groups).

### [Best Mode]

Hereinafter, the present disclosure will be described in more detail through examples. However, these examples are only for describing the present disclosure more specifically, and it will be obvious to those having ordinary knowledge in the art to which the present disclosure belongs that the scope of the present disclosure is not limited by the examples.

### Examples

### Example 1. Experimental animals

7- to 10-week old Balb/c-nude background mice (body weight 20-24 g) were purchased from Orient Bio (Seongnam, Korea). All animal experiments were performed according to the guidelines of the Jeonbuk National University Animal Care and Use Committee. The animals were accommodated under controlled-temperature (21-24 °C) and 12:12-hour light-dark cycle environments and were given free access to water and feed.

### Example 2.1. Induction of differentiation from hESCs into hMSSCs

H9 hESCs (human embryonic stem cells) were purchased from WiCell (Madison, MI, USA). The hESCs were cultured on CF1 mouse embryonic fibroblast (MEF) feeder cells whose cell division was blocked by mitomycin C treatment. A hESC culture medium was prepared with DMEM/F12 (Invitrogen, USA) supplemented with 20% knockout serum replacement (hereinafter, referred to as KSR, Invitrogen, USA), 1 mM glutamine (Invitrogen, USA), 1% nonessential amino acids (Invitrogen, USA), 0.1 mM β-mercaptoethanol (Invitrogen, USA), 0.1% penicillin/streptomycin (Invitrogen, USA) and 15 ng/mL bFGF (R&D Systems, USA).

A medium for inducing differentiation from hESCs into hMSSCs (human musculoskeletal stem cells) (hereinafter, referred to as "MSSC medium") was prepared with the following composition:
1) 250 ng/mL human noggin (Koma Biotech, Korea),
2) 20 ng/mL human LIF (Koma Biotech, Korea),
3) 15 ng/mL basic fibroblast growth factor (FGF; R&D Systems, USA) (FGF2 signaling activator),
4) 3 µM CHIR99021 (Cayman, USA) (Wnt signaling activator),
5) 1 µM PD0325901 (Cayman, USA) (ERK (extracellular signal-regulated kinase) signaling inhibitor),
6) 10 µM SB431542 (Tocris, United Kingdom) (TGF-β/activin/nodal (TGF-β/activin/nodal) signaling inhibitor),
7) Others: 10% knockout serum replacement (Invitrogen, USA), 1% N2 supplement (GIBCO, USA), 2% B27 supplement (GIBCO, USA), 1% nonessential amino acids (GIBCO, USA), 43% DMEM/F12 (GIBCO, USA), 43% Neurobasal (GIBCO, USA), 1 mM glutamine, 0.1 mM β-mercaptoethanol, 0.1% penicillin-streptomycin and 5 mg/mL bovine serum albumin (GIBCO, USA).

The hESCs were treated with a ROCK (Rho-associated coiled-coil kinase) inhibitor (Y-27632, 10 µM, Calbiochem, Germany) and a PKC (protein kinase C) inhibitor (Go6983, 2.5 µM, Sigma, USA) for 24 hours in order to enhance survivability. Then, the hESCs were trypsinized by treating with TrypLE (Life Technologies, USA) and were induced to differentiate into hMSSCs by culturing with the MSSC medium on a culture dish coated with vitronectin + gelatin (1 ng/mL, Sigma, USA) until passage 7. The differentiated MSSCs were identified to be stably identical from passage 5, and the cells cultured for 10 passages were deposited on October 10, 2018 in the Korean Cell Line Bank and were given the accession number KCLRF-BP-00460.

### Example 2.2. Induction of differentiation from iPSCs into hMSSCs

hiPSCs (human induced pluripotent stem cells) were obtained by introducing the OCT4, KLF4, SOX2 and cMYC genes into BJ fibroblasts (ATCC^{®}CRL2522^{™}) using Sendai virus according to the method developed by Hasegawa et al. (Fusaki et al., 2009, PNAS 85, 348-362). The hiPSCs were cultured on CF1 mouse embryonic fibroblast (MEF) feeder cells whose cell division was blocked by mitomycin C treatment. A hiPSC culture medium was prepared with DMEM/F12 (Invitrogen, USA) supplemented with 20% knockout serum replacement (hereinafter, referred to as KSR, Invitrogen, USA), 1 mM glutamine (Invitrogen, USA), 1% nonessential amino acids (Invitrogen, USA), 0.1 mM β-mercaptoethanol (Invitrogen, USA), 0.1% penicillin/streptomycin (Invitrogen, USA) and 15 ng/mL bFGF (R&D Systems, USA).

A medium for inducing differentiation of hiPSCs into hMSSCs (human musculoskeletal stem cells) (hereinafter, referred to as "MSSC medium") was prepared with the following composition:
1) 250 ng/mL human noggin (Koma Biotech, Korea),
2) 20 ng/mL human LIF (Koma Biotech, Korea),
3) 15 ng/mL basic fibroblast growth factor (FGF) (R&D Systems, USA) (FGF2 signaling activator),
4) 3 µM CHIR99021 (Cayman, USA) (Wnt signaling activator),
5) 1 µM PD0325901 (Cayman, USA) (ERK (extracellular signal-regulated kinase) signaling inhibitor),
6) 10 µM SB431542 (Tocris, United Kingdom) (TGF-β/activin/nodal (TGF-β/activin/nodal) signaling inhibitor),
7) Others: 10% knockout serum replacement (Invitrogen, USA), 1% N2 supplement (GIBCO, USA), 2% B27 supplement (GIBCO, USA), 1% nonessential amino acids (GIBCO, USA), 43% DMEM/F12 (GIBCO, USA), 43% Neurobasal (GIBCO, USA), 1 mM glutamine, 0.1 mM β-mercaptoethanol, 0.1% penicillin-streptomycin and 5 mg/mL bovine serum albumin (GIBCO, USA).

The hiPSCs were treated with a ROCK (Rho-associated coiled-coil kinase) inhibitor (Y-27632, 10 µM, Calbiochem, Germany) and a PKC (protein kinase C) inhibitor (Go6983, 2.5 µM, Sigma, USA) for 24 hours in order to enhance survivability. Then, the hiPSCs were trypsinized by treating with TrypLE (Life Technologies, USA) and were induced to differentiate into hMSSCs by culturing with the MSSC medium on a culture dish coated with vitronectin + gelatin (1 ng/mL, Sigma, USA) until passage 7. The differentiated MSSCs were identified to be stably identical from passage 5.

### Example 3. Immunohistochemistry

Samples obtained by injecting the hMSSCs differentiated in Example 2 into the subcutaneous tissue and kidney of Balb/c-nude were fixed overnight at 4 °C in 2% paraformaldehyde (PFA; Wako, Japan). For a sample to investigate differentiation into bone, decalcification was conducted at 4 °C for 2 weeks in PBS (pH 7.2) using 0.4 M EDTA. Then, the samples were dehydrated using ethanol and xylene sequentially, embedded in paraffin or OCT in sucrose, and cut to 5 µm thickness. The specimen was stained with H&E (Cosmobio, Japan).

### Example 4. Fluorescence immunoassay

### "Immunofluorescence staining" was performed as follows.

The samples obtained by injecting the hMSSCs into the subcutaneous tissue and kidney of Balb/c-nude were fixed overnight at 4 °C in 2% paraformaldehyde (PFA; Wako, Japan). All the samples were decalcified with Morse's solution. The samples were embedded in paraffin (Leica Biosystems, Germany) and then cut to 5 µm thickness. After blocking the specimen for 15 minutes in 3% hydrogen peroxide, the samples were incubated at 4 °C overnight with primary antibodies. The primary antibodies treated on the specimen were as follows: mouse monoclonal antibody against HLA class I (Abcam, United Kingdom), goat polyclonal antibody against collagen type II (Santacruz, USA), and antibody against osterix (Abcam, USA). Alexa 555 (Invitrogen, USA) and Alexa 488 (Invitrogen, USA) IgGs were used as secondary antibodies. The immunostained specimen was counterstained with TO-PRO3 (Invitrogen, USA) to visualize nuclei. The fluorescence-labeled cut surface was imaged with the Leica DM 5000 microscope (Leica Microsystems, Germany) or a confocal microscope (LSM510; Carl Zeiss, Germany) and analyzed with the Zen software.

### Example 5. Flow cytometry

After separating the hMSSCs of Examples 2.1 and 2.2 into a single-cell suspension by treating with trypsin/EDTA and blocking nonspecific binding with 2% BSA in PBS, the cells were reacted with monoclonal antibodies against Sca, CD2, CD3, CD4, CD7, CD8, CD10, CD11b, CD14, CD19, CD20, CD31, CD34, CD44, CD45, CD51, CD56, CD73, CD90, CD105, CD146, CD166, CD235a and CD271 (BD Biosciences, USA) in a buffer solution [1XPBS, 1% BSA and 0.01% sodium azide] and then washed. The cells were reacted with Alexa Fluor 488 secondary mouse IgGs (Invitrogen, USA), washed and then analyzed using a flow cytometer (FACStar Plus flow cytometer, BD Biosciences, USA). Normal mouse IgGs (BD Biosciences, USA) were used as a negative control group.

### Example 6.1. Differentiation of human mesenchymal stem cells (hMSCs) and hMSSCs into osteoblasts in vitro

In order to differentiate the hMSSCs of Examples 2.1 and 2.2 into osteoblasts, the cells were cultured in an osteogenic differentiation medium (StemPro^{®} osteogenic differentiation kit, Life Technologies, USA) under the condition of 37 °C and 5% CO₂ for 14 days. Alkaline phosphatase (Roche, Switzerland) staining and alizarin red S (Sigma, USA) staining were conducted to observe osteogenesis. The differentiation of hMSCs (Lonza, Switzerland) into osteoblasts was also compared in the same manner.

### Example 6.2. Differentiation of human mesenchymal stem cells (hMSCs) and hMSSCs into adipocytes in vitro

In order to differentiate the hMSSCs of Examples 2.1 and 2.2 into adipocytes, the cells were cultured in an adipogenic differentiation medium (StemPro^{®} adipogenic differentiation kit, Life Technologies, USA) under the condition of 37 °C and 5% CO₂ for 14 days. Oil red O (Sigma, USA) staining was conducted to observe adipogenesis. The differentiation of hMSCs (Lonza, Switzerland) into adipocytes was also compared in the same manner.

### Example 6.3. Differentiation of human mesenchymal stem cells (hMSCs) and hMSSCs into chondrocytes in vitro

In order to differentiate the hMSSCs of Examples 2.1 and 2.2 into chondrocytes, the cells were resuspended in a chondrogenic differentiation medium (StemPro^{®} chondrogenic differentiation kit, Life Technologies, USA) and then centrifuged. For formation of micromass, the formed pellets were resuspended in a differentiation medium to 1×10⁵ cells/µL and then 5 µL of the cell solution was dropped on the center of a 96-well plate. After incubating the micromass for 2 hours under a high-humidity condition and adding a warmed chondrogenic differentiation medium, incubation was performed in an incubator under the condition of 5% CO₂ and 37 °C. The culture medium was re-feded with 3- to 4-day intervals. After 14 days, the chondrogenic pellets were stained with alcian blue. The differentiation of hMSCs (Lonza, Switzerland) into chondrocytes was also compared in the same manner.

### Example 7.1. Differentiation capacity of hMSSCs into endothelial cells in vitro

It was investigated whether the hMSSCs of Example 2.1 differentiate into endothelial cells (ECs). The hMSSCs were differentiated by culturing with an EC differentiation medium (endothelial growth medium (EGM)-2, Lonza, Walkersville, MD, USA) supplemented with 50 ng/mL VEGF (vascular endothelial growth factor: ProSpec, Rehovot, Israel) and 10 ng/mL bFGF (basic fibroblast growth factor; ProSpec, Rehovot, Israel) for 6 days. The differentiation was confirmed by immunocytochemistry.

### Example 7.2. Differentiation capacity of hMSSCs into skeletal muscle cells in vitro

It was investigated whether the hMSSCs of Examples 2.1 and 2.2 differentiate into skeletal muscle cells. The hMSSCs were differentiated by culturing with a skeletal muscle differentiation medium (DMEM supplemented with 2% B27) for 2 weeks on a Matrigel-coated coverslip. The differentiation was confirmed by immunocytochemistry.

### Example 8. Induction of differentiation of hMSSCs into nerve cells in vitro

For differentiation into nerve cells, the hMSSCs of Example 2.1 were plated on a polyornithine- and laminin-coated culture dish. After 2 days, the culture medium was exchanged with a neural differentiation medium (Neurobasal medium supplemented with 2% B27, 2 mM GlutaMAX and antibiotics). From day 7, 0.5 mM dibutyl cAMP (Sigma, USA) was added every day for 3 days. As a control group, human neural stem cells derived from H9 hESCs (GIBCO, USA) were differentiated into nerve cells in the same manner. The differentiation was confirmed by immunocytochemistry.

### Example 9.1. Differentiation capacity of hMSSCs in mouse kidney

In order to measure the differentiation capacity of the hMSSCs of Example 2.1 in the kidney of mouse, the hMSSCs were cultured with an MSCGM-CD medium (Lonza, Switzerland) for 2-5 passages and collected as single cells. The hMSSCs (2×10⁵ cells) were cultured in an agarose gel well with DMEM + 20% FBS for 2 days to form a cell aggregate, which were transplanted into the kidney capsule of Balb/c nude mouse. Immunohistochemistry and immunofluorescence staining were performed 4 weeks after the transplantation.

### Example 9.2. Differentiation capacity of hMSSCs in mouse subcutaneous tissue

In order to measure the differentiation capacity of the hMSSCs of Example 2.1 in the subcutaneous tissue of mouse, the hMSSCs were cultured with an MSCGM-CD medium (Lonza, Switzerland) for 2-5 passages and collected as single cells. The hMSSCs (2×10⁵ cells) were loaded in fibrin glue (Greenplast^{®}, Green Cross, Korea) to which 1 µg/mL hyaluronic acid (Sigma, USA) was added and then transplanted into the subcutaneous tissue of Balb/c nude mouse. Immunohistochemistry and immunofluorescence staining were performed 4 weeks after the transplantation.

### Example 10.1. Bone formation test using hMSCs

For analysis of osteogenesis of hMSCs in a thighbone fracture model, hMSCs (Lonza, Switzerland) were cultured with an MSCGM-CD medium (Lonza, Switzerland) for 7 passages, collected as single cells and then absorbed into a collagen membrane (SK Bioland, Korea) cut to a size of 1 mm × 1 mm. After perforating one tibia of a 6-week-old Balb/c- nude mouse by about 1 mm using a drill (Bosch Professional, Germany), the hMSCs absorbed in the collagen membrane were inserted into the fracture area of the mouse. The mouse was anesthetized every 2 weeks and micro-CT (Skyscan 1076, Antwerp, Belgium) images were obtained for the fracture area. Immunohistochemistry and immunofluorescence staining were performed 6 weeks later.

### Example 10.2. Bone formation test using hMSSCs

For analysis of osteogenesis of hMSSCs in a thighbone fracture model, the hMSSCs of Example 2.1 were cultured with an MSCGM-CD medium (Lonza, Switzerland) for 2-5 passages, collected as single cells and then absorbed into a collagen membrane (SK Bioland, Korea) cut to a size of 1 mm × 1 mm. After perforating one tibia of a 6-week-old Balb/c- nude mouse by about 1 mm using a drill (Bosch Professional, Germany), the hMSSCs absorbed in the collagen membrane were inserted into the fracture area of the mouse. The mouse was anesthetized every 2 weeks and micro-CT (Skyscan 1076, Antwerp, Belgium) images were obtained for the fracture area. Immunohistochemistry and immunofluorescence staining were performed 6 weeks later.

### Example 11. Micro-CT

The bone formed in the kidney in which the hMSSCs were transplanted in Example 10.1 was scanned by micro-CT (Skyscan 1076, Antwerp, Belgium) to obtain 3D CT (computed tomography) images. Then, the data were digitalized with a frame grabber and the resulting images were transmitted to a computer using the Comprehensive TeX Archive Network (CTAN) topographic reconstruction software.

### Example 12. Measurement of SCX, Runx2 and MYH9 mRNA expression levels

RNAs were extracted from the transplant of the hMSSCs of Example 2.1 in the kidney using 500 µL of Trizol (Life Technologies, USA) according to the manufacturer's protocol. After treating the transplant derived from hMSSCs in the kidney with DNAse (RQ1 DNase, Promega, USA), 500 ng of RNAs were reversely transcribed to cDNAs using oligo-d(T) and random hexamers according to the Superscript III RT (Life Technologies, USA) first-strand cDNA synthesis protocol. qRT-PCR was conducted on the StepOne Plus PCR cycler (Applied Biosystems) using Sybr green (Applied Biosystems, Foster City, CA). mRNA expression data were analyzed using the ΔΔCT method and normalized with glyceraldehyde-3-phosphate dehydrogenase (GAPDH) for gene detection. The primers necessary for the qRT-PCR were purchased from Qiagen (USA). As a control group, RNAs were extracted from hMSSCs and qRT-PCR was conducted in the same manner.

### Example 13. Confirmation of differentiation of hMSSCs derived from hESCs

### Senescence marker

Differentiation from hESCs into hMSSCs was induced as described in Example 2 and the morphological change of the induced hMSSCs was observed. The result is shown in FIG. 1A. As seen from FIG. 1A, it was confirmed that the undifferentiated single H9 hESCs were differentiated into cells with fibroblast morphology within 7 passages. They grew with similar morphologies for 10 passages or longer, from passage 7 until passage 17, and showed positive response to staining with the senescence marker β-gaiactosidase since passage 19, suggesting that aging was progressed.

### Confirmation of pluripotency marker by immunofluorescence method

The expression of pluripotency markers in the hMSSCs after 7 passages or longer since the induction from the hESCs was observed by the immunofluorescence method, and the result is shown in FIG. 1B. For comparison, the expression of the pluripotency markers in H9 hESCs was investigated by the immunofluorescence method.

As seen from FIG. 1B, the H9 hESCs were positive for all of OCT4, NANOG, SOX2 and LIN28, suggesting that they have pluripotency. In contrast, the hMSSCs derived from the H9 hESCs were negative for OCT4, NANOG, SOX2 and LIN28.

### Confirmation of pluripotency, ectodermal, mesodermal and endodermal markers through RNA sequencing

The expression of pluripotency, ectodermal, mesodermal and endodermal markers in hESCs, hMSCs and hMSSCs at passages 7 and 17 was investigated through RNA sequencing, and the result is shown in FIG. 1C. The expression of the mRNAs of the pluripotency markers TDGF, NANOG, POU5F1, SOX2, DPPA4, LEFTY1, GDF3, etc. was confirmed in the H9 hESCs (hESC-1, hESC-2). In contrast, for the hMSSCs derived from the H9 hESCs, the expression of the pluripotency marker DPPA4 was observed but the expression of the pluripotency markers TDGF, NANOG, POU5F1, LEFTY1 and GDF3 was not observed. The expression level of DPPA4 was comparable to that in the H9 hESCs.

The expression of DPPA4 was not observed in the human mesenchymal stem cells. In addition, the hMSSCs were positive for the ectodermal marker NES, were positive for most mesodermal markers except for DES and the early mesodermal markers T and nodal, and were negative for most endodermal markers. In particular, NES was not expressed in the mesenchymal stem cells.

### Confirmation of mesenchymal stem cell markers through expression of cell surface antigens

The expression of surface antigens of hMSSCs was measured as shown in FIG. 1D. When the expression of mesenchymal stem cell-specific cell surface antigens was investigated, the expression of the mesenchymal stem cell markers CD44, CD51, CD73, CD105, CD146 and CD166 was observed in the hMSSCs, but the expression of the mesenchymal stem cell markers CD90 and CD271 was not observed in the hMSSCs. In addition, the expression of the blood-associated cell surface markers CD2, CD3, CD7, CD8, CD11b, CD14, CD19, CD20, CD31, CD34 and CD56 was not observed, but the expression of the pre-B cell marker CD10 was observed.

### Confirmation of other cell-specific markers

The expression of tissue-specific markers of different lineages was analyzed to investigate the characteristics of hMSSCs as shown in FIG. 1E. The mesodermal marker alpha smooth muscle actin (a-SMA), the neuroectodermal marker Pax6, the myogenic satellite marker Pax7, the intestinal stem cell marker LGR5, etc. were expressed, whereas the chondrocyte marker SOX9, the myoblast marker MyoD, etc. were not expressed. This suggests that the hMSSCs are progenitor cells prior to differentiation into chondrocytes and muscle cells.

### Example 14. Differentiation capacity of hMSSCs in vitro

In-vitro osteogenesis, chondrogenesis and adipogenesis were tested for hMSCs and the hMSSCs of Example 2.1 (Example 6), and the result is shown in FIG. 2A. From FIG. 2A, it was confirmed that the hMSCs were differentiated into bone, cartilage and adipose in vitro. Meanwhile, the hMSSCs were differentiated into bone and cartilage but were hardly differentiated into adipose under the same condition for the hMSCs in vitro. In other words, it was confirmed that there was a functional difference from mesenchymal stem cells.

### Differentiation capacity into skeletal muscle

It was investigated whether the hMSSCs of Example 13 have the potential to differentiate into skeletal muscle.

The hMSSCs were cultured for 2 weeks in a skeletal muscle differentiation medium (DMEM containing 2% B27) on a Matrigel-coated coverslip, and then immunofluorescence assay was performed for the skeletal muscle marker MYH9. The result is shown in FIG. 2B. C2C12 cells were used as a positive control group. As shown in FIG. 2B, it was confirmed that the skeletal muscle-specific marker MYH9 was expressed when the hMSSCs were cultured in the skeletal muscle differentiation medium, suggesting that the hMSSCs have the potential to differentiate into skeletal muscle.

### Differentiation capacity into endothelial cells

It was investigated whether the hMSSCs of Example 13 have the potential to differentiate into endothelial cells.

The hMSSCs were cultured for 6 days in an EC differentiation medium (endothelial growth medium (EGM)-2; Lonza, Walkersville, MD) supplemented with 50 ng/mL VEGF (vascular endothelial growth factor: ProSpec, Rehovot, Israel) and 10 ng/mL bFGF (basic fibroblast growth factor; ProSpec), and then immunofluorescence assay was performed for the endothelial cell markers CD31 and VE-cadherin. The result is shown in FIGS. 2C and 2D. HUVECs were used as a positive control group for endothelial cell differentiation. As shown in FIG. 2C and FIG. 2D, the expression of CD31 and VE-cadherin was not observed in the hMSSCs, suggesting that the hMSSCs lack the potential to differentiate into endothelial cells. In contrast, the expression of the markers was observed in the control group HUVECs.

### Differentiation capacity into nerve cells

hMSSCs were incubate for 7 days in a neural differentiation medium (Neurobasal medium containing 2% B27, 2 mM GlutaMAX and antibiotics) and then cultured for 3 days while adding 0.5 mM dibutyl cAMP (Sigma) every day. Then, immunofluorescence assay was performed for the nerve cell differentiation marker MAP2. The result is shown in FIG. 2E. NSCs (neuronal stem cells) were used as a positive control group for nerve cell differentiation. As shown in FIG. 2E, the cell morphology of the NSCs was changed to that of nerve cells and the expression of the nerve cell-specific marker MAP2 was observed, suggesting that the cells were differentiated into nerve cells. In contrast, the hMSSCs showed no change in cell morphology and the expression of MAP2 was not observed, suggesting that they lack the potential to differentiate into nerve cells.

Although the hMSSCs were positive for the ectodermal marker NES as confirmed in Example 13, they did not differentiate into nerve cells. It was confirmed that the hMSSCs can differentiate into the mesoderm, more specifically into bone, cartilage and muscle.

### Example 15. Confirmation of differentiation of hMSSCs into bone, cartilage, muscle, adipose and tendon in vivo

In order to measure the differentiation potential of the hMSSCs induced in the same manner as in Example 2 in vivo, the hMSSCs were transplanted into the kidney (Example 9.1) and subcutaneous tissue (Example 9.2) of an immunodeficient mouse. After transplanting the hMSSCs into the kidney of the mouse and staining tissues with H&E 3-4 weeks later, immunofluorescence staining was performed for bone-, muscle-, adipose-, tendon- and ligament-specific markers and the cell nuclei were counterstained with TO-PRO3. The result is shown in FIG. 3A and FIG. 3B.

FIG. 3A shows the images obtained 4 weeks after culturing the hMSSCs in an MSCGM-CD medium (Lonza, Switzerland) for 2-5 passages and transplanting them into the kidney. The H&E staining result shows that muscle, adipose, tendon and ligament were formed in the kidney (FIG. 3A-a). As a result of confirming the differentiated muscle tissue derived from hMSSCs, the differentiation into skeletal muscle was observed but the differentiation into smooth muscle was not observed. In contrast, when human MSCs were transplanted under the same condition, muscle, adipose, tendon, etc. were not formed at all (data not shown). In addition to, for immunohistochemistry analysis confirmed that each differentiated tissue was positive for the muscle marker phospho-myosin light chain (pMLC), the adipose marker PPAR-gamma (PPAr), the tendon or ligament marker scleraxis (SCX), etc., and was also positive for the human cell marker hLA (human leukocyte antigen). In this reason, it can be seen that the transplanted hMSSCs were differentiated into muscle, adipose and tendon (or ligament) cells (This is contrary to the in-vitro test result showing no differentiation into adipose.) (FIG. 3A-b).

FIG. 3B-a a micro-CT scan image showed that hard tissue, i.e., bone, was formed at the hMSSCs transplanted site into the kidney.

FIGS. 3B-b and c show a result of confirming bone formation by H&E staining and pentachrome immunohistochemistry. It can be seen that the transplanted hMSSCs were differentiated into bone in the kidney capsule.

FIG. 3B-dshows the result of immunohistochemical assay for the transplanted site. It was confirmed that the cells in the tissue were positive for the human cell marker hLA (human leukocyte antigen), the bone markers Osx (osterix), Runx2, DMP1, OCN (osteocalin), etc. and the blood vessel marker vWF, confirming that bone was formed. Therefore, it can be seen that the transplanted hMSSCs were differentiated into bone.

FIG. 3C shows that the hMSSCs transplanted into the mouse subcutaneous tissue after being loaded in fibrin glue to which hyaluronic acid was added were differentiated into chondrocytes. The formation of cartilage was confirmed through H&E staining and toluidine blue staining.

Taken together, it was confirmed that the hMSSCs of the present disclosure can differentiate into cartilage, muscle, tendon (or ligament) and bone at the transplanted site and have superior differentiation capacity.

### Example 16. Confirmation of fracture recovery effect of hMSSCs

In order to investigate the fracture recovery effect of hMSSCs induced in the same manner as in Example 2, bone formation test was performed as in Example 10. The result is shown in FIG. 4A and FIG. 4B.

It was confirmed that bone was formed at the fracture site about 6 weeks after hMSC transplantation using the femur fracture model. However, the results showed that the bone formation site was positive for the bone marker Runx2, whereas it was negative for the human cell marker hLA. This suggests that the bone formation was not caused by hMSCs but the cells of the mouse itself formed the bone (FIG. 4A). In contrast, hMSSCs were transplanted under the same condition, bone was formed at the fracture site about 6 weeks later, and it was confirmed that the osteogenic site was positive for Runx2 and positive for the human cell marker hLA. Thus, It was confirmed that bone was formed by differentiation of hMSSCs (FIG. 4B).

### Example 17. Induction of differentiation from hiPSCs into hMSSCs and characterization of induced hiPSCs

hiPSC (human induced pluripotent stem cells) were prepared by reprogramming embryonic IMR90 fibroblasts by sendai virus-mediated overexpression of OCT4, KLF4, SOX2 and MYC according to the protocol developed by Hasegawa et al. (Fusaki et al., 2009).

hMSSCs (hereinafter, iPS-hMSSCs) were obtained by inducing hMSSCs from hiPSCs as in Example 2. The expression level of the pluripotency markers Oct4, Nanog, Sox2 and Lin28 in the iPS-hMSSCs was investigated by immunofluorescence assay and RT-PCR. The result is shown in FIG. 5A.

As seen from FIG. 5A, it was confirmed that iPS cells were positive for all of OCT4, NANOG, SOX2 and LIN28, suggesting that they have pluripotency. In contrast, the iPS-hMSSCs were negative for all the pluripotency markers OCT4, NANOG, SOX2 and LIN28.

FIG. 5B shows a result of measuring the expression of surface antigens for the iPS-hMSSCs. When the expression of mesenchymal stem cell-specific cell surface antigens was investigated, it was confirmed that, among the mesenchymal stem cell markers, CD44, CD51, CD73, CD105, CD146 and CD166 were expressed in the iPS-hMSSCs but CD90 and CD271 were not expressed in the iPS-hMSSCs. In addition, the pre-B cell marker CD10 was expressed whereas the blood-associated cell surface markers CD2, CD3, CD7, CD8, CD14, CD20 and CD56 were not expressed.

Also, the osteogenesis, chondrogenesis and adipogenesis of the iPS-hMSSCs were investigated in the same manner as in Example 14, and the result is shown in FIG. 5C. As seen from FIG. 5C, it was confirmed that the hMSSCs derived from the hiPSCs were differentiated into bone and cartilage in vitro but were hardly differentiated into adipose.

In addition, the iPS-hMSSCs were cultured for 2 weeks in a skeletal muscle differentiation medium (DMEM containing 2% B27) on a Matrigel-coated coverslip and then immunofluorescence assay was performed for the skeletal muscle marker MYH9. The result is shown in FIG. 5D. C2C12 cells were used as a positive control group. As seen from FIG. 5D, it was confirmed that the hMSSCs derived from the hiPSCs have the potential to differentiate into skeletal muscle.

Taken together, it was confirmed that the hMSSCs derived from the hiPSCs have the same characteristics as the hMSSCs derived from the hECSs, suggesting that hMSSCs can be obtained using hiPSCs instead of hECSs.

### Example 18. Differentiation capacity of hMSSCs derived from hiPSCs in vivo

### Transplantation into kidney

After transplanting the hMSSCs of Example 17 into mouse kidney, tissue was stained with H&E 3-4 weeks later. It was confirmed that typical muscle, adipose and tendon (or ligament) were formed in the kidney. As a result of immunohistochemical assay, the transplanted site was positive for the muscle marker phospho-myosin light chain (pMLC), the adipose marker PPAR-gamma (PPAr), the tendon or ligament marker scleraxis (SCX), etc. and was also positive for the human cell marker hLA (human leukocyte antigen). Also, it was positive for the bone markers Osx (osterix), Runx2, DMP1, OCN (osteocalin), etc. As a result, it was confirmed that the hMSSCs induced from the iPSCs can differentiate into muscle, adipose, tendon (or ligament) and bone.

### Transplantation into subcutaneous tissue

When the hMSSCs of Example 17 were transplanted into mouse subcutaneous tissue after being loaded in fibrin glue to which hyaluronic acid was added, it was confirmed through H&E staining and toluidine blue staining that the hMSSCs can differentiate into cartilage.

### Example 19. Comparison of differentiation capacity of noggin-containing MSSC medium and conditioned medium-containing CM medium

The differentiation capacity of a medium (hereinafter, referred to as "CM medium") obtained by replacing human noggin (Life Technologies), which is the ingredient 1) of the seven ingredients of the MSSC medium of Example 2, with conditioned medium (culture supernatant obtained after culturing CF1 cells for 24 hours using complete medium wherein DMEM/F12 is replaced with knockout DMEM (knockout DMEM supplemented with 20% knockout serum replacement (Invitrogen, USA), 1 mM glutamine, 1% nonessential amino acids (Invitrogen, USA), 0.1 mM β-mercaptoethanol, 0.1% penicillin-streptomycin and 5 mg/mL bovine serum albumin) (the remaining ingredients 2)-7) are the same) was compared with that of the MSSC medium.

Noggin is generally used to maintain the characteristics of hESCs during culturing (Chaturvedi G, Simone PD, Ain R, Soares MJ, Wolfe MW. Noggin maintains pluripotency of human embryonic stem cells grown on Matrigel. Cell Prolif. 2009 Aug; 42(4): 425-33). Contrarily to the previously known mechanism, it significantly increased the tendency toward the mesoderm. As can be seen from Table 1, the tendency of osteogenic differentiation was increased 10 times or greater when noggin was contained, as compared to when the CM medium was used.

**[Table 1]**

| Differentiation tendency of MSSC medium vs. CM medium (number of differentiations out of 20 observations) | | | | |
|---|---|---|---|---|
| Induction medium | Bone | Muscle | Tendon | Adipose |
| CM medium | 1/20 | 20/20 | 2/20 | 2/20 |
| Noggin-containing medium | 15/20 | 20/20 | 10/20 | 12/20 |

In addition, the expression level of CD44 was compared for the two media. After inducing differentiation using the CM-containing medium (CM medium) and the noggin-containing medium (MSSC medium), the expression level of CD44 was measured in the same manner as in Example 6. As a result, it was confirmed that the expression level of CD44 was increased remarkably when the noggin-containing MSSC medium was used as compared to when the CM medium was used (FIG. 6).

During osteogenic differentiation, the formation of endochondral bone occurs necessarily after chondrogenesis. CD44 is known to play an essential role in chondrogenesis (Wu SC, Chen CH, Chang JK, Fu YC, Wang CK, Eswaramoorthy R, Lin YS, Wang YH, Lin SY, Wang GJ, Ho ML: Hyaluronan initiates chondrogenesis mainly via cd44 in human adipose-derived stem cells. J Appl Physiol (1985) 2013; 114: 1610-1618). As a result, it was confirmed that use of the MSSC medium rather than the CM medium is suitable for osteogenic differentiation.

When hMSSCs were transplanted into the kidney, the cells differentiated by the hMMSC medium showed 1-2 weeks faster differentiation as compared to the cells differentiated by the CM medium. The difference in differentiation speed when the CM medium was used and when the hMMSC medium was used is shown in Table 2.

**[Table 2]**

| Differentiation speed of MSSC medium vs. CM medium (increase in mRNA level as compared to before transplantation of hMSSCs) | | | | | |
|---|---|---|---|---|---|
| mRNA | | Week 1 | Week 2 | Week 3 | Week 4 |
| MYH9 | CM | 1.3 ± 0.1 | 2.1 ± 0.1 | 5.1 ± 0.3 | 12.5 ± 3.1 |
| MYH9 | MSSC | 2.2 ± 0.3 | 4.4 ± 0.4 | 20.1 ± 3.1 | 23.1 ± 3.4 |
| Runx2 | CM | 1.2 ± 0.3 | 1.8 ± 0.3 | 3.6 ± 0.3 | 6.5 ±3.1 |
| Runx2 | MSSC | 2.1 ± 0.2 | 4.3 ± 0.3 | 7.1 ± 0.3 | 13.3 ± 3.1 |
| SCX | CM | 1.3 ± 0.2 | 2.3 ± 1.2 | 5.2 ± 1.3 | 10.7 ± 2.2 |
| SCX | MSSC | 2.1 ± 0.2 | 4.7 ± 1.5 | 12.1 ± 0.3 | 16.5 ± 2.9 |

### Example 20. Comparison of synergistic effect for combinations of ingredients of MSSC medium

The differentiation capacity of the MSSC medium of Example 2 was compared for the cases where one of the ingredients 1)-6) was missing and the case where all the ingredients were contained. As a result, it was confirmed that differentiation into cartilage (alcian blue) or bone (ALP and alizarin red S) was not achieved well when one of the ingredients 1)-6) was absent (FIG. 7, Table 3).

**[Table 3]**

| Comparison of differentiation capacity of MSSC medium vs. medium with one ingredient missing | | | | | | | |
|---|---|---|---|---|---|---|---|
| Ingredient s of MSSC medium | 6 factors | TGF-β/activin/ nodal signaling activator (-) | hLIF (-) | ERK signaling inhibitor (-) | Wnt signaling activator (-) | Noggin (-) | FGF-2 signaling activator (-) |
| 1) Noggin | ○ | ○ | ○ | ○ | ○ | × | ○ |
| 2) LIF | ○ | ○ | × | ○ | ○ | ○ | ○ |
| 3) FGF-2 signaling activator | ○ | ○ | ○ | ○ | ○ | ○ | × |
| 4) Wnt | ○ | ○ | ○ | ○ | × | ○ | ○ |
| signaling activator | | | | | | | |
| 5) ERK signaling inhibitor | ○ | ○ | ○ | × | ○ | ○ | ○ |
| 6) TGF-β/acti vin/nodal signaling inhibitor | ○ | × | ○ | ○ | ○ | ○ | ○ |
| Remarks | Muscle (including adipose), cartilage and osteogenic differentiation achieved as desired | Muscle and cartilage differentiation not achieved | Cartilage differentiation not achieved | Cartilage and bone differentiation inhibited | Bone differentiation inhibited | MSSCs not induced | MSSCs not induced |

### Example 21. Induction of selective differentiation of hMSSCs into muscle, ligament, cartilage and bone in vivo

In order to measure the possibility of selective differentiation of hMSSCs, hMSSCs were transplanted into the kidney (kidney capsule) or subcutaneous tissue of Balb/c nude mouse by pretreating with various growth factors such as connective tissue growth factor, transforming growth factor, insulin, fibroblast growth factor, etc. and then preparing into a cell aggregate (4×10⁵ cells) or mixing with a cell carrier. 3-6 weeks after the transplantation, tissue was analyzed after removing the transplanted cells.

### Example 22. Confirmation of induced differentiation of hMSSCs into muscle

For investigation of the selective differentiation capacity of the hMSSCs induced in the same manner as in Example 2 into muscle in vivo, hMSSCs were mixed with fibrin glue to which heparin was added and then transplanted into the subcutaneous tissue of an immunodeficient mouse. 7 weeks after the transplantation of hMSSCs, tissue was stained with H&E and the STEM101 antibody (Takara, Japan) binding to the human cell nucleus marker in order to identify whether the differentiated tissue was derived from the hMSSCs. In addition, the differentiated tissue was immunofluorescence staining with an antibody against the muscle marker MyoD (Thermo Fisher Scientific Inc., USA) in order to identify muscle cells. The cell nuclei were counterstained with DAPI. The result is shown in FIG. 8. As shown in FIG. 8, it was confirmed that the hMSSCs were differentiated into muscle only.

### Example 23. Confirmation of induced differentiation of hMSSCs into tendon or ligament

For investigation of the selective differentiation capacity of the hMSSCs induced in the same manner as in Example 2 into tendon or ligament in vivo, hMSSCs were pretreated with 50 ng/mL connective tissue growth factor and 25 µg/mL ascorbic acid for 2 days in an MSCGM-CD medium (Lonza, Switzerland) and the pretreated cells (1×10⁶ cells) were transplanted into the subcutaneous tissue of an immunodeficient mouse after mixing with 100 µL of Matrigel (BD Sciences, USA). 5 weeks after the transplantation of hMSSCs, tissue was stained with H&E and an antibody against hLA binding to the human cell nucleus marker (Abcam, United Kingdom) in order to identify whether the differentiated tissue was derived from the hMSSCs. In addition, the differentiated tissue was immunofluorescence staining with an antibody against the tendon or ligament marker SCX (Antibodies-Online, USA) in order to identify whether the differentiated tissue was tendon or ligament. The result is shown in FIG. 9. As shown in FIG. 9, it was confirmed that the hMSSCs were differentiated into tendon or ligament only.

### Example 24. Confirmation of induced differentiation of hMSSCs into bone

For investigation of the selective differentiation capacity of the hMSSCs induced in the same manner as in Example 2 into bone in vivo, hMSSCs were pretreated with 5 µg/mL insulin, 25 µg/mL ascorbic acid and 1 µg/mL hyaluronic acid for 2 days in a medium supplemented with 10% knockout serum replacement (Invitrogen, USA), 1% N2 supplement (GIBCO, USA), 2% B27 supplement (GIBCO, USA), 43% DMEM/F12 (GIBCO, USA) and 43% Neurobasal (GIBCO, USA), prepared into a cell aggregate and then transplanted into the subcutaneous tissue of an immunodeficient mouse. 5 weeks after the transplantation of hMSSCs, tissue was stained with H&E. In addition, the differentiated tissue was immunofluorescence staining for the cartilage- and bone-specific markers collage type II (colli) and osterix (osx) and cell nuclei were counterstained with DAPI. The result is shown in FIG. 10. As shown in FIG. 10, it was confirmed that the hMSSCs were differentiated into bone only through endochondral ossification.

### Example 25. Confirmation of induced differentiation of hMSSCs into tooth

For investigation of the selective differentiation capacity of the hMSSCs induced in the same manner as in Example 2 into tooth in vivo, hMSSCs were prepared into a cell aggregate, coated with mouse dental epithelial cells and then transplanted into the subcutaneous tissue of an immunodeficient mouse. 6 weeks after the transplantation of hMSSCs, tissue was stained with H&E. In addition, the differentiated tissue was immunofluorescence staining for the human cell-specific marker hLA and cell nuclei were counterstained with To-PRO-3. The result is shown in FIG. 11. As shown in FIG. 11, it was confirmed that the hMSSCs were differentiated into tooth only. In FIG. 11A, K indicates kidney tissue, T indicates tooth tissue, and B indicates bone tissue. FIG. 11B shows the micro-CT image of the kidney of FIG. 11A. FIG. 11C shows the image of the H&E-stained tissue. FIG. 11D shows the fluorescence image obtained by staining with the antibody against human leukocyte antigen (hLA). To-PRO-3 shows the result of counterstaining cell nuclei.

### Example 25. Therapeutic effect of hMSSCs on ligament damage

In order to evaluate the possibility of treating damaged ligament with hMSSCs induced in the same manner as in Example 2 in vivo, the posterior thoracolumbar ligament of an immunodeficient mouse was damaged by cutting along a perpendicular direction and then a cell aggregate prepared by mixing hMSSCs (2×10⁶ cells) with 10 µL of fibrin glue was transplanted into the damaged site. 6 weeks after the transplantation of the hMSSCs, tissue was stained with H&E and immunofluorescence staining was performed for the human cell-specific marker hLA and the tendon- or ligament-specific marker SCX. The result is shown in FIG. 12. Cell nuclei were counterstained with To-PRO-3. As seen from FIG. 12, it was confirmed that the broken ligament was connected as the hMSSCs were differentiated.

### Example 26. Therapeutic effect of hMSSCs on arthritis

In order to evaluate the possibility of treating damaged cartilage with hMSSCs induced in the same manner as in Example 2 in vivo, destabilization of medial meniscus (DMM) surgery was performed on the left joint of 10-week-old C57BL/6 mice and hMSSCs (3×10⁵) were injected into the articular cavity 4-6 weeks later. Experimental groups were divided into a sham control group (Sham), a group to which DMM was performed and the cells were injected after thawing, spinning down and resuspending in PBS (hMSSC-PBS), and a group to which the cells contained in a freezing solution were injected without any treatment (hMSSC-Sol). At week 8, samples were acquired, fixed, decalcified in 10% EDTA solution for 4 weeks, sectioned (5 µm) and then stained with safranin O. The severity of arthritis was evaluated by the method proposed by the Osteoarthritis Research Society International (OARSI). As shown in FIG. 13, the arthritis index (OA score) was increased in the DMM group as compared to the Sham group, suggesting that osteoarthritis was induced well. In the hMSSC-injected groups, the severity of arthritis was decreased to half or lower, and there was no difference between the PBS and Sol groups. At week 6 after the injection of hMSSCs, the severity of arthritis was decreased as compared to at week 4, but there was no statistical difference. It was confirmed that the hMSSCs are therapeutically effective even when they are stored by freeze-drying and administered immediately after thawing (FIG. 13).

Although the specific exemplary embodiments of the present disclosure have been described in detail, it will be obvious to those having ordinary knowledge in the art that they are merely specific examples and the scope of the present disclosure is not limited by them. It is to be noted that the substantial scope of the present disclosure is defined by the appended claims and their equivalents.

### [Accession Number]

Depository agency: Korean Cell Line Bank
Accession number: KCLRFBP00460
Date of deposition: 20181010

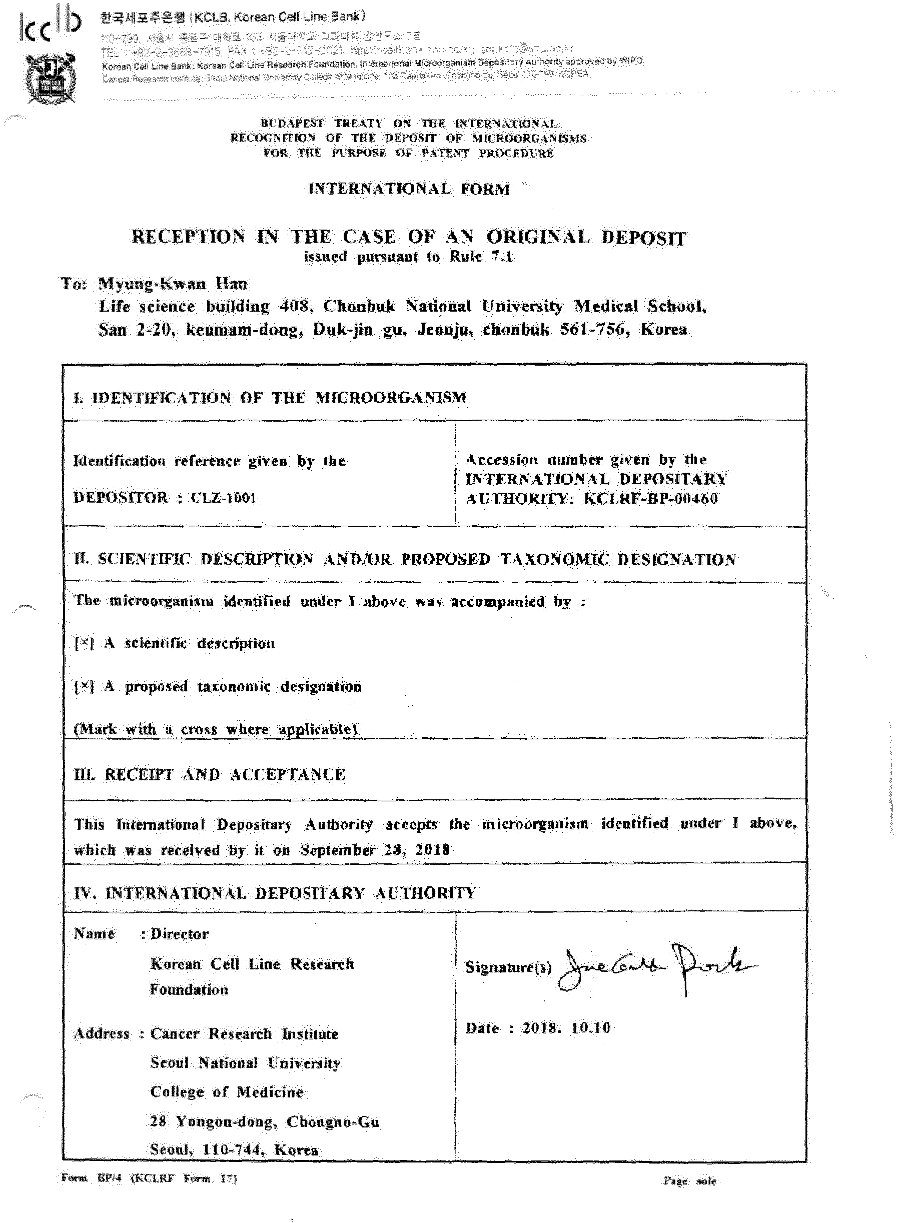
한국세포주은행 (KCLB, Korean Cell Line Bank)
110-799, 서울시 종로구 대학로 103 서울대학교 의과대학 암연구소 7층
TEL : +82-2-3668-7915, FAX : +82-2-742-0021, http://cellbank.snu.ac.kr, snukclb@snu.ac.kr
Korean Cell Line Bank: Korean Cell Line Research Foundation, International Microorganism Depository Authority approved by WIPO
Cancer Research Institute, Seoul National University College of Medicine, 103 Daehak-ro, Chongno-gu, Seoul 110-799, KOREA

BUDAPEST TREATY ON THE INTERNATIONAL
RECOGNITION OF THE DEPOSIT OF MICROORGANISMS
FOR THE PURPOSE OF PATENT PROCEDURE

INTERNATIONAL FORM

RECEPTION IN THE CASE OF AN ORIGINAL DEPOSIT
issued pursuant to Rule 7.1

To: Myung-Kwan Han
Life science building 408, Chonbuk National University Medical School,
San 2-20, keumam-dong, Duk-jin gu, Jeonju, chonbuk 561-756, Korea

I. IDENTIFICATION OF THE MICROORGANISM
Identification reference given by the
DEPOSITOR : CLZ-1001 | Accession number given by the
INTERNATIONAL DEPOSITARY
AUTHORITY: KCLRF-BP-00460
II. SCIENTIFIC DESCRIPTION AND/OR PROPOSED TAXONOMIC DESIGNATION
The microorganism identified under I above was accompanied by :
[×] A scientific description
[×] A proposed taxonomic designation
(Mark with a cross where applicable)
III. RECEIPT AND ACCEPTANCE
This International Depositary Authority accepts the microorganism identified under I above,
which was received by it on September 28, 2018
IV. INTERNATIONAL DEPOSITARY AUTHORITY
Name : Director
Korean Cell Line Research
Foundation

Address : Cancer Research Institute
Seoul National University
College of Medicine
28 Yongon-dong, Chongno-Gu
Seoul, 110-744, Korea | Signature(s)

Date : 2018. 10.10

Form BP/4 (KCLRF Form 17)
Page sole

## Claims

1. A medium composition inducing of selective differentiation into only muscle from musculoskeletal stem cells (MSSCs) that can be differentiated into bone, tooth, cartilage, tendon, ligament, muscle and adipose, wherein the medium composition comprises heparin and the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

2. A pharmaceutical composition for preventing or treating a musculoskeletal disease, comprising the medium composition for inducing differentiation according to claim 1 and musculoskeletal stem cells (MSSCs) as active ingredients.

3. A method for inducing selective differentiation from musculoskeletal stem cells (MSSCs) into only muscle, comprising a step of treating heparin in the musculoskeletal stem cells that can be differentiate into bone, tooth, cartilage, tendon, ligament, muscle and adipose, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

4. A medium composition for selectively inducing differentiation into only tendon or ligament from musculoskeletal stem cells (MSSCs) that can be differentiated into bone, tooth, cartilage, tendon, ligament, muscle and adipose wherein the medium composition comprises connective tissue growth factor (CTGF) and ascorbic acid and the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

5. A pharmaceutical composition for preventing or treating a musculoskeletal disease, comprising musculoskeletal stem cells (MSSCs) pretreated with the medium composition for inducing differentiation according to claim 4 as active ingredients.

6. A method for inducing selective differentiation from musculoskeletal stem cells (MSSCs) into only tendon or ligament, comprising a step of treating connective tissue growth factor (CTGF) and ascorbic acid in musculoskeletal stem cells that can be differentiated into bone, tooth, cartilage, tendon, ligament, muscle and adipose, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

7. A medium composition for selectively inducing differentiation into only bone from musculoskeletal stem cells (MSSCs) that can be differentiated into bone, tooth, cartilage, tendon, ligament, muscle and adipose, wherein the medium composition comprises insulin, hyaluronic acid and ascorbic acid and the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

8. A pharmaceutical composition for preventing or treating a musculoskeletal disease, comprising musculoskeletal stem cells (MSSCs) pretreated with the medium composition for inducing differentiation according to claim 7 as active ingredients.

9. A method for inducing selective differentiation from musculoskeletal stem cells (MSSCs) into only bone, comprising a step of treating insulin, hyaluronic acid and ascorbic acid in the musculoskeletal stem cells that can be differentiated into bone, tooth, cartilage, tendon, ligament, muscle and adipose, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

10. A medium composition for selectively inducing differentiation into only tooth from musculoskeletal stem cells (MSSCs) that can be differentiated into bone, tooth, cartilage, tendon, ligament, muscle and adipose, wherein the medium composition comprises dental epithelial cells and the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

11. A method for inducing selective differentiation from musculoskeletal stem cells (MSSCs) into only tooth, comprising a step of coating with dental epithelial cells for musculoskeletal stem cells that can be differentiated into bone, tooth, cartilage, tendon, ligament, muscle and adipose, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

12. The medium composition according to claim 1, 4, 7 or 10, wherein the musculoskeletal stem cells further have the following characteristics:
e) negative for the mesenchymal stem cell marker CD90;
f) negative for the mesenchymal stem cell marker CD271;
g) positive for the pluripotency marker DPPA4;
h) negative for the mesodermal markers T and nodal;
i) positive for the neuroectodermal marker Pax6;
j) positive for the intestinal stem cell marker LGR5;
k) negative for the chondrocyte marker SOX9; or
l) negative for the myoblast marker MyoD.

13. A method for treating damaged tendon or ligament, comprising a step of administering musculoskeletal stem cells (MSSCs) that can differentiate into bone, tooth, cartilage, tendon, ligament, muscle and adipose to a damaged tendon or ligament area of a subject, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

14. A method for treating arthritis, comprising a step of administering musculoskeletal stem cells (MSSCs) that can differentiate into bone, tooth, cartilage, tendon, ligament, muscle and adipose into the articular cavity of a subject, wherein the musculoskeletal stem cells have the following characteristics:
a) positive for the ectodermal marker nestin (NES);
b) positive for the myogenic satellite marker Pax7;
c) positive for the mesodermal marker α-SMA; and
d) negative for the pluripotency marker LIN28.

15. The method according to claim 3, 6, 9, 11, 13 or 14, wherein the musculoskeletal stem cells further have the following characteristics:
e) negative for the mesenchymal stem cell marker CD90;
f) negative for the mesenchymal stem cell marker CD271;
g) positive for the pluripotency marker DPPA4;
h) negative for the mesodermal markers T and nodal;
i) positive for the neuroectodermal marker Pax6;
j) positive for the intestinal stem cell marker LGR5;
k) negative for the chondrocyte marker SOX9; or
l) negative for the myoblast marker MyoD.
